(19)

Europäisches Patentamt

European Patent Office

Office européen des brevets

(11) **EP 1 909 103 A1**

(12) **EUROPEAN PATENT APPLICATION**
published in accordance with Art. 153(4) EPC

(43) Date of publication:
**09.04.2008 Bulletin 2008/15**

(21) Application number: **06781077.0**

(22) Date of filing: **13.07.2006**

(51) Int Cl.:
*G01N 33/52* (2006.01)          *G01N 21/78* (2006.01)
*G01N 33/543* (2006.01)

(86) International application number:
**PCT/JP2006/314001**

(87) International publication number:
**WO 2007/007849 (18.01.2007 Gazette 2007/03)**

(84) Designated Contracting States:
**DE FR GB**

(30) Priority: **14.07.2005 JP 2005205587**

(71) Applicant: **MATSUSHITA ELECTRIC INDUSTRIAL CO., LTD.**
**Osaka 571-8501 (JP)**

(72) Inventors:
• **KAWAMATA, Ryoko**
**c/o Matsushita Elec. Ind. Co.**
**2-1-61 Shiromi, Chuo-ku, Osaka-shi, Osaka 540-6207 (JP)**

• **TAKAHASHI, Mie**
**Matsushita Elec. Ind. Co**
**2-1-61, Shiromi, Chuo-ku, Osaka 540-6207 (JP)**

(74) Representative: **Eisenführ, Speiser & Partner**
**Patentanwälte Rechtsanwälte**
**Postfach 10 60 78**
**28060 Bremen (DE)**

(54) **ANALYZER AND ANALYZING METHOD**

(57)      In an analysis device for measuring a target substance in a liquid sample, highly precise measurement cannot be realized because reliability of measurement is degraded due to influences of properties of the liquid sample and an analysis element.

There is provided an analysis device comprising a signal measurement unit for measuring a signal based on a reaction of the target substance in the liquid sample, a parameter collection unit for collecting a parameter that indicates a degree of influence on a measurement error from the liquid sample developed on a channel on the analysis element, an algorithm holding unit for previously holding an algorithm comprising a relationship among the parameter, the signal, and a true value of the target substance, and an arithmetic processing unit for arithmetically processing an analysis value of the target substance from the signal on the basis of the parameter, and the arithmetic processing unit reads out the algorithm, and obtains an analysis value of the target substance with the measurement error of the target substance being corrected, on the basis of the parameter obtained in the parameter collection unit by using the read algorithm.

Fig.1

60 analysis device

**EP 1 909 103 A1**

**Description**

TECHNICAL FIELD

The present invention relates to an analysis device for analyzing a target substance in a liquid sample, and more particularly, an analysis device which can reliably correct a measurement error of the target substance according to a degree of influence on the measurement error due to the properties of the liquid sample and the analysis element during detection of the target substance in the liquid sample, and an analysis method performed by the analysis device.

BACKGROUND ART

In recent years, as home health care and local health care such as hospitals and clinics have been well developed and further early diagnosis and clinical examination of high urgency have been increased, there has been an increasing demand for an analysis device with which a person, even if he/she is not a specialist in clinical examination, can execute highly precise measurement easily and speedily. Therefore, a compact analysis device for POCT (Point of Care Testing) which can perform highly reliable measurement in short time without requiring complicated operations has been highlighted.

The POCT is a generic term for examinations which are performed in "places close to patients" such as doctor's offices of practitioners and specialists, hospital wards, and clinics for ambulant patients, and this POCT attracts attention as a method that greatly contributes to improvement in the quality of medical care, by which a doctor immediately judges a test result, performs a speedily treatment, and performs up to monitoring for the process of curing as well as catamnestic monitoring. An examination by such compact analysis device can reduce costs for conveyance of analyte and facilities in contrast to an examination in a central laboratory, thereby realizing a reduction in the total examination cost. So, the POCT market is rapidly extended in the Unites States where rationalization of hospital management is progressing, and it is expected that the POCT will be a growing market from a global perspective including in Japan.

A dry analysis element represented by an immunochromatography sensor can analyze a target substance in a liquid sample such as blood or urine to be a target of measurement by only a simple operation such as dropping the liquid sample onto the analysis element without requiring adjustment of a reagent, and it is very useful for easily and speedily analyzing the target substance in the liquid sample, and therefore, a lot of dry analysis elements are put to practical use as a representative of the POCT in these days. Furthermore, higher analysis precision is demanded from the market in addition to that anybody can perform measurement anytime and anywhere.

However, the above-mentioned dry analysis element has the following drawbacks. Since liquid samples have individual differences in their properties such as viscosity, amount of each component, and foreign substance, and an analysis method using such dry analysis element is likely to be affected by these properties, and therefore, it is difficult to obtain a highly precise measurement result as compared with a large-scale analysis device which can eliminate these factors. So, analysis devices which can eliminate the above-mentioned factors that reduce the reliability of measurement have conventionally been developed by many makers.

For example, when the liquid sample is blood, there is hematocrit as a typical individual difference, and when a target substance in the blood is analyzed by the dry analysis element, the analysis is dominantly influenced by a hematocrit value in the blood. So, there have been reported various analysis devices which detect the hematocrit value and correct the concentration of the target substance according to the value by various methods (refer to Patent Document 1 to Patent Document 3).

Any of the above-mentioned methods can reliably correct a measurement error that occurs when a sample in which a hematocrit value in whole blood deviates from a normal range is measured, and it is applicable to quantitative determination for the target substance in the whole blood. However, in any method, correction is performed for only the influence of the hematocrit value, and influences due to the properties other than the hematocrit value such as the viscosity of the liquid sample or the foreign substances cannot be corrected.

On the other hand, the factor that reduces the reliability of measurement is not restricted to the above-mentioned hematocrit value, but the property such as the viscosity of the liquid sample, the analysis environment, and the deactivation of the reagent can be the factor. There has been proposed an analysis method which considers the influences on the analysis result by the factors that reduce the reliability in measurement, such as the property of the liquid sample, the analysis environment, and the deactivation of the reagent, as well as the hematocrit value. For example, there is proposed a method in which a signal that is generated when, after a specific binding reaction, a signal substance generator which relates to the specific binding and generates a signal substance diffuses in a channel and reaches detectors is measured by plural detectors that are disposed in the flow direction, and a difference or a ratio of signal modulation is obtained in each detector, thereby performing an arithmetic processing so as to minimize influences on the analysis result due to nonspecific factors other than the target substance in the liquid sample (refer to Patent Document 4).

Patent Document 1: Japanese Patent Publication No.3586743
Patent Document 2: Japanese Published Patent Application No.2000-262298
Patent Document 3: Japanese Published Patent Application No.2001-91512
Patent Document 4: Japanese Published Patent Application No.Hei.8-75748

DISCLOSURE OF THE INVENTION

PROBLEMS TO BE SOLVED BY THE INVENTION

As described above, the methods disclosed in Patent Documents 1 to 3 can reliably correct a measurement error that occurs when measuring a sample in which a hematocrit value in whole blood deviates from a normal range, and therefore, it is applicable to quantitative determination for a target substance in whole blood. However, the methods disclosed in Patent Documents 1 to 3 perform such correction for only an influence due to a hematocrit value when the liquid sample is blood, but cannot perform correction for a result that also excludes influences due to the properties derived from the liquid sample other than the hematocrit value, such as the viscosity of the liquid sample, the foreign substances in the sample, and the like, resulting in very poor measurement precision.

On the other hand, in the analysis method discloses in Patent Document 4 which considers the influences due to the factors that reduce the reliability in measurement such as the property of the liquid sample, it is premised that the plural detectors are equally subjected to the influences. However, plural stages of reactions are performed until reaching generation of a signal that becomes an indicator for minimization of the influences on the analysis result, and thereby differences in reaction states are likely to occur, and therefore, it is difficult to reliably minimize the influences. Further, the method disclosed in Patent Document 4 requires the plural detectors, and thereby the construction of the analysis device should be complicated, resulting in a problem that the complexity might be a factor that deteriorates the measurement precision, and the device is expensive in cost.

Furthermore, the above-mentioned methods cannot be introduced in a chromatography sensor which is required to be operable by anyone, anytime, and anywhere, in view of operability and construction, resulting in a lack of versatility.

Further, it is impossible for the conventional methods to automatically identify a liquid sample from among different kinds of liquid samples such as whole blood, blood plasma, urine, and the like. Therefore, it is necessary to previously indicate what liquid sample is used in a chromatography sensor, and a wrong liquid sample might be used by mistake depending on the field where it is used, and thereby an accurate result cannot be obtained. In recent years, there has frequently occurred a problem that throughout education for handlers of diagnostic agents in the fields is indispensable even through the diagnostic agents are for the POCT.

The present invention is made to solve the above-described problems and has for its object to provide an analysis device and an analysis method which can identify what liquid sample is used, and can easily obtain an analysis value with a measurement error of a target substance in the liquid sample being corrected according to a degree of influence on the measurement error due to the properties of the liquid sample and the analysis element, with which anybody can perform easy, speedy, and highly-precise measurement anytime and anywhere.

MEASURES TO SOLVE THE PROBLEMS

In order to solve the above-described problems, according to the present invention, there is provided an analysis device for developing a liquid sample in a channel on an analysis element and measuring a target substance in the liquid sample, which analysis device comprises a signal measurement unit for measuring a signal based on a reaction of the target substance in the liquid sample on the channel; a parameter collection unit for collecting a parameter which indicates a degree of influence on a measurement error of the target substance from the liquid sample developed on the channel; an algorithm holding unit for previously holding an algorithm comprising a relationship among the parameter, the signal, and a true value of the target substance; and an arithmetic processing unit for arithmetically processing an analysis value of the target substance from the signal on the basis of the parameter; wherein the arithmetic processing unit reads out the algorithm from the algorithm holding unit, and obtains, using the read algorithm, an analysis value of the target substance with the measurement error of the target substance being corrected on the basis of the parameter obtained in the parameter collection unit.

Therefore, it is possible to correct the measurement error of the target substance which is caused by the properties of the liquid sample and the analysis element, thereby providing a simple, speedy, and highly-accurate analysis device.

The measurement error described here is a degree of deviation of a measurement value of the target substance from a true value of the target substance, which deviation occurs due to influences of the properties of the liquid sample and the analysis element. Further, the algorithm comprises a relationship among the parameter, the signal, and the true value of the target substance. That is, the algorithm comprises a relationship between the parameter and the measurement error of the target substance, or a relationship between the signal and the true value of the target substance, which enables calculation of an analysis value of the target substance that is corrected based on the parameter. For example, the algorithm may include a numerical formula for performing an arithmetic operation on the basis of the parameter, plural numerical formulae which are prepared for selecting a degree of correction based on the parameter, and other arbitrarily methods.

Further, the parameter is numerically converted information relating to development of the liquid sample, and it may include, for example, a developing speed, a developing distance, and a developing time of the liquid sample. However, there is no problem in adopting other information such that a change of absorbance in a background that does not contribute to the reaction.

Furthermore, the influence of the properties of the liquid sample and the analysis element indicates an arbitrary influence that causes a measurement error, such as different kinds of liquid samples when the liquid sample is any of whole blood, blood plasma, urine, and bacterial cell suspending solution, or excess or shortage of the additive amount of the liquid sample, or variation in the content degree of cells in the liquid sample when the liquid sample is whole blood or bacterial cell suspending solution, or a change in the property due to the length of the storage period of the analysis element or a difference in the storage environment of the analysis element.

Further, in the analysis device of the present invention, the arithmetic processing unit includes a measurement value calculation unit for calculating a measurement value of the target substance in the liquid sample from the signal obtained in the signal measurement unit, and a measurement value correction unit for correcting the measurement value of the target substance so as to minimize the measurement error of the target substance, thereby obtaining an analysis value of the target substance; and the measurement value correction unit reads out an algorithm comprising a relationship between the parameter and the measurement error of the target substance from the algorithm holding unit, and corrects the measurement value of the target substance which is obtained in the measurement value calculation unit on the basis of the parameter obtained in the parameter collection unit by using the read algorithm, thereby obtaining an analysis value of the target substance.

Therefore, it is possible to obtain an analysis value of the target substance by correcting the measurement value of the target substance so as to minimize the measurement error of the target substance which is caused by the properties of the liquid sample and the analysis element, thereby providing a simple, speedy, and highly-accurate analysis device.

Further, in the analysis device of the present invention, the analysis element includes a sample application part for applying the liquid sample onto the channel, a marker reagent part which holds a marker reagent that reacts with the target substance so that the marker reagent can be eluted by development of the liquid sample, and a sample immobilization part which immobilizes and holds a reagent that comprehends the degree of the reaction between the target substance and the marker reagent.

Therefore, an accurate analysis value of the target substance in the liquid sample can be easily and speedily obtained.

Further, in the analysis device of the present invention, the parameter is any of a developing speed, a developing time, and a developing distance which are obtained when the liquid sample is developed on the channel.

Therefore, the developing state of the liquid sample which is influenced by a difference in the properties of the liquid sample and the analysis device can be detected more accurately and quantitatively. By using this developing state, a speedy and simple analysis device having higher accuracy and versatility can be obtained.

Further, in the analysis device of the present invention, at least one of the signal measurement unit and the parameter collection unit uses electromagnetic radiation.

Therefore, it becomes possible to detect the degree of minute modulation in the signal on the channel, thereby providing an analysis device having higher precision and accuracy.

Further, in the analysis device of the present invention, the analysis element is a dry type analysis element.

Therefore, the analysis device is easy to carry because the entire analysis element is a dry carrier, and further, it is easy to handle because there is no necessity of strictly controlling the storage environment and storage condition, thereby providing a speedy, simple, and highly-accurate analysis device anytime and anywhere.

Further, in the analysis device of the present invention, the signal measurement unit measures a signal based on a reaction that is derived from an antigen-antibody reaction.

Since the antibody can be artificially created, the target that can be detected in this analysis device is diversified, and thereby measurement of various kinds of target substances can be carried out. Further, an analysis device of higher accuracy can be provided by making use of a specific reaction of the antibody.

Further, in the analysis device of the present invention, the analysis element is an immunochromatography sensor.

Therefore, it is possible to realize a high accuracy which prevents a user from performing erroneous judgment, and a simple operation which can be used by anyone, anytime and anywhere.

Further, in the analysis device of the present invention, the analysis element is a one-step immunochromatography sensor.

Therefore, it is possible to provide a simple, speedy, and highly-accurate analysis device which does not require complicated operations such as pretreatment and washing, even using a immunoassay method.

Further, in the analysis device of the present invention, the channel comprises a monolayer or multilayer porous material.

Therefore, it becomes possible to reliably hold the reagent in the channel and develop the liquid sample, thereby providing a simple, speedy, and highly-accurate analysis device in analysis of the liquid sample.

Further, in the analysis device of the present invention, the algorithm holding unit holds a correction formula for correcting the measurement value of the target substance on the basis of the parameter; and the measurement value correction unit reads out the correction formula from the algorithm holding unit, and corrects the measurement value of the target substance using the read correction formula and the parameter, thereby obtaining an analysis value of the target substance.

Therefore, the measurement error of the target substance due to the properties of the liquid sample and the analysis element can be easily corrected, and thereby a more accurate analysis value of the target substance can be speedily and easily obtained.

Further, in the analysis device of the present invention, the algorithm holding unit holds a plurality of the correction formulae; the analysis device further includes an algorithm selection unit for selecting any of the plural correction formulae that are stored in the algorithm holding unit on the basis of the measurement value of the target substance; and the measurement value correction unit corrects the measurement value of the target substance using the correction formula selected by the algorithm selection unit and the parameter, thereby obtaining an analysis value of the target substance.

Therefore, the measurement error of the target substance due to the properties of the liquid sample and the analysis element can be corrected using a correction formula that is selected according to the measurement value of the target substance from among the plural correction formulae, and thereby a more accurate analysis value of the target substance can be speedily and easily obtained.

Further, in the analysis device of the present invention, the algorithm holding unit holds a plurality of calibration curves for obtaining an analysis value of the target substance with the measurement error of the target substance being corrected, from the signal obtained in the signal measurement unit; the analysis device further includes an algorithm selection unit for selecting any of the plural calibration curves that are stored in the algorithm holding unit on the basis of the parameter; and the arithmetic processing unit obtains an analysis value of the target substance with the measurement error of the target substance being corrected, from the signal by using the calibration curve selected by the algorithm selection unit and the parameter.

Therefore, the assumed measurement error of the target substance due to the properties of the liquid sample and the analysis device can be minimized by using a calibration curve that is selected according to the parameter from among the plural calibration curves, and thereby a more accurate analysis value of the target substance can be obtained speedily and easily.

Further, in the analysis device of the present invention, the arithmetic processing unit obtains an analysis value of the target substance with the measurement error of the target substance due to an influence of the viscosity of the liquid sample, or the additive amount of the liquid sample, or the passage time after fabrication of the analysis element being corrected.

Therefore, an accurate analysis value of the target substance in the liquid sample can be obtained regardless of whatever property or additive amount the liquid sample has. Further, it is possible to avoid an influence of deterioration of the analysis element due to passage time after manufacturing.

Further, according to the present invention, there is provided an analysis method for developing a liquid sample in a channel on an analysis element, and measuring a target substance in the liquid sample, which method comprises a parameter collection step of collecting a parameter which indicates a degree of influence on a measurement error of the target substance, from the liquid sample developed on the channel; a signal measurement step of measuring a signal based on a reaction of the target substance in the liquid sample on the channel; and an arithmetic processing step of reading out an algorithm from an algorithm holding unit which previously holds an algorithm comprising a relationship among the parameter, the signal, and a true value of the target substance, and obtaining, using the read algorithm, an analysis value of the target substance with the measurement error of the target substance being corrected on the basis of the parameter obtained in the parameter collection unit.

Therefore, the measurement error of the target substance due to the properties of the liquid sample and the analysis element can be easily corrected, thereby obtaining a highly accurate analysis value.

Further, in the analysis method of the present invention, the arithmetic processing step includes a measurement value calculation step of calculating a measurement value of the target substance in the liquid sample from the signal obtained in the signal measurement step, and a measurement value correction step of correcting the measurement value of the target substance to obtain an analysis value of the target substance.

Therefore, it is possible to obtain a more accurate analysis value by correcting the measurement value of the target substance so as to remove the measurement error of the target substance due to an influence of the properties of the liquid sample and the analysis element.

EFFECTS OF THE INVENTION

According to the present invention, when performing measurement of a target substance in a liquid sample, a parameter which indicates a degree of influence on a measurement error of the target substance due to the properties of the liquid sample and the analysis element is collected from the liquid sample that is developed on a channel on the analysis element, and the measurement error of the target substance in the liquid sample is corrected according to the parameter. Thereby, a highly accurate analysis value of the target substance can be obtained easily and speedily.

BRIEF DESCRIPTION OF THE DRAWINGS

Figure 1 is a conceptual diagram of an analysis device according to a first embodiment.
Figure 2 is an exploded view illustrating an analysis element utilizing chromatography according to the first embodiment.
Figure 3 is a perspective view illustrating the analysis element utilizing chromatography according to the first embodiment.
Figure 4 is a diagram illustrating a signal measurement unit for measuring a signal derived from a marker reagent in a reagent immobilization part on the analysis element according to the first embodiment.
Figure 5 is a diagram schematically illustrating a result of reading of color reaction on reagent immobilization parts I and II on the analysis element, which result is obtained by the signal measurement unit according to the first embodiment.
Figure 6 is a diagram illustrating a parameter collection unit for collecting parameters from a liquid sample that is developed on the analysis element according to the first embodiment.
Figure 7 is a diagram illustrating a liquid sample developing state with passage of time on the analysis element according to the first embodiment.
Figure 8 is a diagram illustrating an optical change before and after arrival of a liquid sample to a detection part on the analysis element according to the first embodiment.
Figure 9 is a conceptual diagram for explaining a correction method according to the first embodiment.
Figure 10 is a diagram illustrating a relationship between the developing speed of a liquid sample and the degree of deviation from a true value in a first example of the present invention.
Figure 11 is a diagram illustrating CV values before and after correction with respect to a hematocrit value difference factor in the first example.
Figure 12 is a distribution diagram illustrating deviation degrees from the true value before and after correction with respect to the hematocrit value difference factor in the first example.
Figure 13 is a distribution diagram illustrating deviation degrees from the true value before and after correction with respect to a total protein concentration difference factor in a second example of the present invention.
Figure 14 is a distribution diagram illustrating deviation degrees from the true value before and after correction with respect to a liquid sample additive amount shortage factor in a third example of the present invention.
Figure 15 is a distribution diagram illustrating deviation degrees from the true value before and after correction with respect to a hematocrit value/total protein concentration difference factor according to a fourth example of the present invention.
Figure 16 is a distribution diagram illustrating degrees of deviation from the true value which are obtained with correction and without correction using plural calibration curves in a fifth example of the present invention.
Figure 17 is a diagram illustrating a relationship between the hematocrit value and the developing speed of the liquid sample in a case where the liquid sample according to the first embodiment is whole blood.
Figure 18 is a diagram illustrating a relationship between the total protein concentration and the developing speed of the liquid sample in a case where the liquid sample according to the first embodiment is blood plasma.
Figure 19 is a diagram illustrating a relationship between the additive value of the liquid sample and the developing speed of the liquid sample according to the first embodiment.
Figure 20 is a flowchart of CRP quantification according to the first example of the present invention.
Figure 21 is a diagram illustrating the developing speeds of the respective liquid samples according to a sixth example of the present invention.
Figure 22(a) is a conceptual diagram of hCG quantification according to the sixth example of the present invention.

Figure 22(b) is a conceptual diagram of hCG quantification according to the sixth example of the present invention.

Figure 22(c) is a flowchart of hCG quantification according to the sixth embodiment of the present invention.

Figure 23 is a distribution diagram illustrating the degrees of deviation from the true value before and after correction in a case where the liquid sample adopted in the sixth example is urine.

Figure 24 is a distribution diagram illustrating the degrees of deviation from the true value before and after correction in a case where the liquid sample adopted in the sixth example is blood plasma.

Figure 25 is a distribution diagram illustrating the degrees of deviation from the true value before and after correction in a case where the liquid sample adopted in the sixth example is whole blood.

DESCRIPTION OF REFERENCE NUMERALS

| | |
|---|---|
| 1 | developing layer (channel) |
| 2 | marker reagent part |
| 3 | reagent immobilization part I |
| 4 | reagent immobilization part II |
| 5 | liquid impermeable sheet member |
| 6 | file space (sample application part) |
| 7 | open part |
| 8 | substrate |
| 9 | fine space formation member |
| 10 | cell component contraction agent |
| 13 | detection part |
| 14 | liquid sample |
| 20 | signal measurement unit |
| 21,31 | irradiation unit |
| 22,32 | light-receiving unit |
| 30 | parameter collection unit |
| 40 | algorithm holding unit |
| 50 | measurement value correction unit |
| 60 | analysis device |
| 70 | measurement value calculation unit |
| 80 | algorithm selection unit |
| 90 | arithmetic processing unit |
| 100 | analysis element |
| 110 | liquid sample identification algorithm |
| 120 | measurement value correction algorithm |
| 130 | liquid sample identification algorithm selection |
| 140 | measurement value correction algorithm selection |
| 150 | calibration curve |
| 160 | calibration curve selection |

BEST MODE TO EXECUTE THE INVENTION

Hereinafter, embodiments of analysis devices according to the present invention will be described in detail with reference to the drawings. However, the embodiments described below are merely examples, and the present invention is not restricted thereto.

(Embodiment 1)

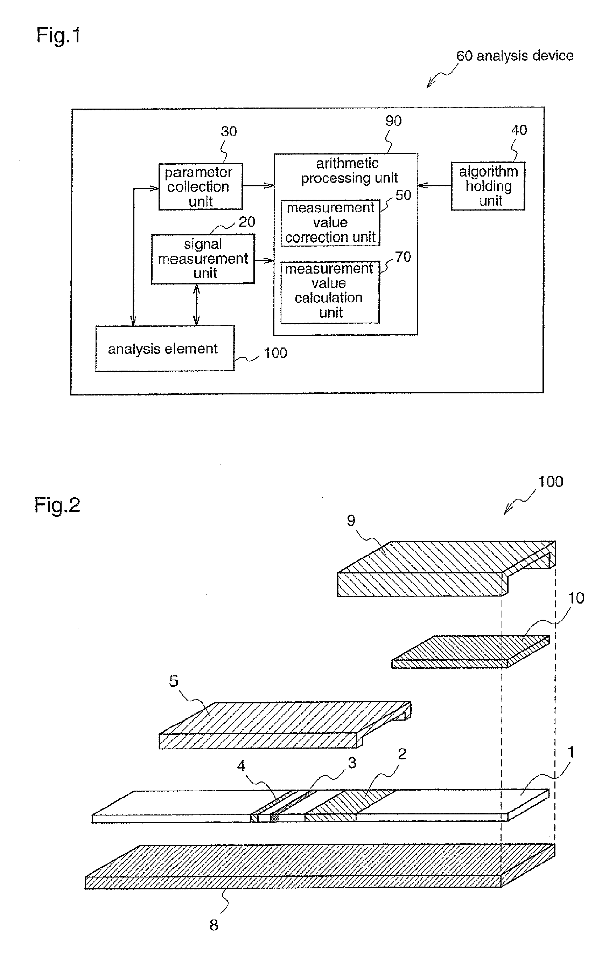

Figure 1 is a conceptual diagram illustrating the construction of an analysis device according to a first embodiment. The analysis device 60 of the first embodiment is provided with a signal measurement unit 20 for measuring a signal based on a reaction of a target substance in a liquid sample on an analysis element 100, which liquid sample is developed in a channel on the liquid sample 100, a parameter collection unit 30 for collecting a parameter which indicates a degree of influence of the properties of the liquid sample and the analysis device 60 on a measurement error of the target substance, from the liquid sample that is developed on the analysis element, an algorithm holding unit 40 for previously holding an algorithm comprising a relationship among the parameter, the signal, and a true value of the target substance, and an arithmetic processing unit 90 for reading the algorithm from the algorithm holding unit 40 according to the parameter obtained by the parameter collection unit 30, and arithmetically processing an analysis value of the target substance with the measurement error being corrected from the signal measured by the signal measurement unit 20, using the read algorithm. The arithmetic processing unit 90 is provided with, according to need, a measurement value calculation unit 70 for calculating a measurement value of the target substance in the liquid sample from the signal obtained in the signal measurement unit 20, and a measurement value correction unit 50 for correcting the measurement value of the target substance so as to minimize the measurement error of the target substance, thereby obtaining an analysis value of the target substance.

The measurement error of the target substance indicates a degree of deviation of the measurement value of the target substance that is calculated from the signal measured by the signal measurement unit 20, from the true value of the target substance, which deviation is caused by influences of the properties of the liquid sample and the analysis element 100. Further, the algorithm comprises a relationship between the parameter and the measurement error of the target substance or a relationship between the signal and the true value of the target substance, and it enables calculation of an analysis value of the target substance which is corrected based on the parameter. For example, the algorithm may include a numerical formula for performing an arithmetic operation on the basis of the parameter, and plural numerical formulae which are prepared for selecting a degree of correction based on the parameter, and further, other arbitrarily methods. Further, the parameter is numeric-converted information relating to development of the liquid sample, and it may include, for example, a developing speed, a developing distance, and a developing time of the liquid sample. However, there is no problem in adopting information other than mentioned above, such as a change of absorbance in a background that does not contribute to the reaction.

Further, while in figure 1 the signal measurement unit 20 and the parameter collection unit 30 are separately shown, the signal measurement unit 20 and the parameter collection unit 30 may be constituted as a single unit. Further, while in figure 1 the algorithm holding unit 40 and the arithmetic processing unit 90 are separately shown, the algorithm holding unit 40 may be included in the arithmetic processing unit 90.

First of all, the analysis element 100 according to the first embodiment will be described.

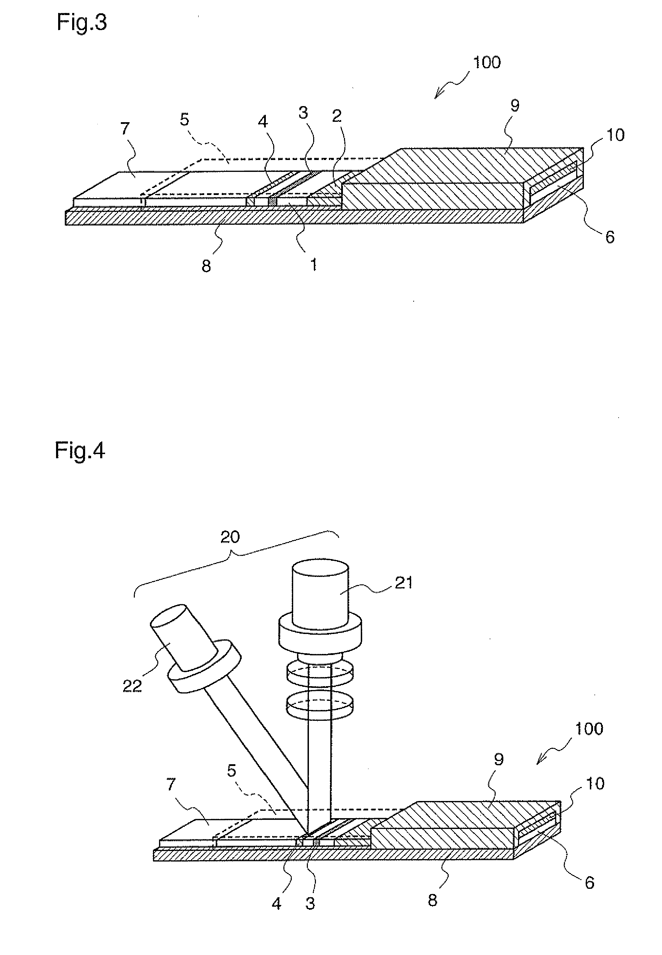

Figures 2 and 3 are diagrams illustrating the analysis element utilizing chromatography according to the first embodiment. More specifically, figure 2 is an exploded view of the analysis element 1, and figure 3 is a perspective view of the analysis element 1.

In figure 2, reference numeral 1 denotes a developing layer serving as a channel, and it comprises nitrocellulose. The material used for the developing layer 1 is not restricted to nitrocellulose, and an arbitrary porous material such as filter paper, nonwoven fabric, membrane, fabric, or fiberglass may be adopted so long as it can be wetted by a liquid sample, and can develop the liquid sample. Alternatively, the developing layer 1 may be formed of a hollow capillary tube, and in this case, a plastic material is used. Further, the developing layer 1 may be composed of a monolayer or multilayer porous material, and a monolayer porous material is adopted in this first embodiment. The "monolayer" means that it is composed of a single layer, and the "multilayer" means that multiple layers are arranged parallel or vertically, and a liquid sample applied to a first layer in the multiple layers can successively migrate to the respective layers.

Reference numeral 2 denotes a marker reagent part in which a gold colloid marker antibody against the target substance in the liquid sample is held in its dry state so as to be dissolvable by development of the liquid sample. The marker reagent is obtained by labeling an antibody with a marker such as gold colloid, and it is used as a means for detecting bindings in reagent immobilization parts 3 and 4 which are described later. The gold colloid is merely an example, and any material may be arbitrarily selected according to need from among, for example, metal or nonmetal colloid particle, enzyme, protein, dye, fluorescent dye, and colored particle such as latex.

Reference numerals 3 and 4 denote a reagent immobilization part I and a reagent immobilization part II, respectively, which are antibodies that can specifically react against the target substance in the liquid sample. Both the antibodies are bound to the target substance with epitopes different from that of the marker reagent, and they are immobilized in their dry states. Further, the antibody used for the reagent immobilization part I and the antibody used for the reagent immobilization part II have different affinities to the target substance in the liquid sample.

The antibodies used for the reagent immobilization part I and the reagent immobilization part II have only to form complexes with the marker reagent and the target substance, and therefore, the epitopes or the affinities of the antibodies for the target substance may be the same or different from each other. Further, the two antibodies may have the same epitope while they have different affinities.

Furthermore, while in figure 2 two reagent immobilization parts are provided, the number of the reagent immobilization parts is not necessarily two, and one or more reagent immobilization parts may be arbitrarily selected according to the purpose. The shapes of the reagent immobilization parts on the developing layer are not necessarily lines, and any shapes such as spots, characters, or keys, may be arbitrarily selected. While in figure 2 the reagent immobilization part 13 and the reagent immobilization part II4 are spatially apart from each other, these parts are not necessarily apart from each other, and may be connected with each other so as to be apparently a single line.

Reference numeral 5 denotes a liquid impermeable sheet, and it comprises a transparent tape in this first embodiment. The liquid impermeable sheet 5 has a configuration to hermetically cover the developing layer 1, except a portion contacting a fine space 6 serving as a sample application part, and an end portion to which the liquid sample reaches.

As described above, the liquid impermeable sheet 5 that covers the developing layer 1 has a function of blocking spot-application of the liquid sample onto a part other than the sample application as well as preventing contamination from the outside, and furthermore, it prevents the liquid sample from being evaporated while it is developed, whereby the liquid sample always passes through the reagent immobilization parts 3 and 4 and the marker reagent part 2 which are reaction parts on the developing layer, and thus reactions with the target substance in the liquid sample can be efficiently carried out in the reaction parts. The contamination from the outside indicates an accidental contact of the liquid sample to the reaction parts on the developing layer, or a direct touch of a patient's hand or the like to the developing layer. Preferably, a transparent material is used as the liquid impermeable sheet 5 that covers the developing layer 1, and at least portions of the sheet 5 covering the reagent immobilization parts 3 and 4 are desired to be transparent because these parts 3 and 4 measure a signal.

Further, when more accurate measurement is required, an upper portion of the developing layer 1, particularly including the marker reagent part 2 and the reagent immobilization parts 3 and 4, may be hermetically sealed and, further, side surfaces parallel to the liquid sample developing direction may be hermetically sealed as well.

Reference numeral 7 denotes an open part in the developing layer 1, and reference numeral 8 denotes a substrate which supports the developing layer 1. The substrate 8 comprises a liquid impermeable sheet such as a PET film, and it may be any of transparent, semitransparent, and nontransparent. However, it is desirable to adopt a transparent material when measuring transmitted light, and a nontransparent material when measuring reflected light. The materials may include synthetic resins such as ABS, polystyrene, and polyvinyl chloride, and liquid impermeable materials such as metal and glass.

The substrate 8 has a function of reinforcing the developing layer 1, and a function of blocking the sample when a sample having a risk of infection such as blood, saliva, or urine is used. Further, when there is a possibility that the developing layer 1 becomes to have optical transparency when it is wetted, the substrate 8 may have an effect of blocking light.

Reference numeral 9 denotes a fine space formation member, having a function of forming a space into which the liquid sample flows due to a capillary phenomenon, and comprising laminated transparent PET films. The fine space formation member 9 also has a function of avoiding contamination of the exterior by the liquid sample when handling the analysis element after application of the liquid sample. The fine space formation member 9 may comprise a synthetic resin material such as ABS, polystyrene, or polyvinyl chloride, or a liquid impermeable material such as metal or glass. While the fine space formation member 9 is desired to be transparent or semitransparent, it may be nontransparent and colored, and it may be composed of an arbitrary nontransparent material.

Reference numeral 6 denotes a fine space. The fine space 6 is formed by the fine space formation member 9, and serves as a sample application part into which the liquid sample is introduced by a capillary phenomenon. The fine space 6 is connected with the developing layer 1, and development of the liquid sample onto the developing layer 1 can be started by introducing the liquid sample into the fine space 6.

Next, a method for measuring the target substance in the liquid sample by the analysis device 60 according to the first embodiment will be described with reference to figures 2 and 3.

When the liquid sample is brought into contact with the fine space 6, the liquid sample naturally flows into the fine space 6 by a capillary phenomenon without the necessity of mechanical operation, and the liquid sample is developed on the developing layer (channel) 1. Whether the flow amount of the liquid sample is sufficient or not can be checked through the fine space formation member 9. When it is necessary to ensure a predetermined additive amount of the liquid sample, the additive amount can be precisely restricted by setting the volume of the fine space 6 to a predetermined volume. Further, when more than a predetermined amount of the liquid sample is required, a structure which holds a volume larger than the required amount is adopted to realize this.

A cell component contraction reagent 10 is stored in the fine space 6. The cell component contraction reagent 10 is a reagent to be provided when cell components are included in the liquid sample, and therefore, it is not especially needed when using a liquid sample including no cell components. Further, the cell component contraction reagent 10 may be any reagent having an effect of contracting cells, such as inorganic compounds including potassium chloride, sodium chloride, and sodium phosphate salt, or amino acids such as glycine and glutamic acid, or imino acids such as proline, or sugars such as glucose, sucrose, and trehalose, or sugar alcohols such as glucitole. A system including such cell component contraction reagent 10 is especially effective when whole blood is used as a liquid sample.

The liquid sample introduced into the fine space 6 is developed from the contact portion of the fine space 6 and the developing layer 1 onto the developing layer 1. When the liquid sample reaches the marker reagent part 2, dissolution of the marker reagent is started. When the target substance exists in the liquid sample, development is promoted while the marker reagent and the target substance react with each other, and the liquid sample reaches the reagent immobilization part 13. When the target substance exists in the liquid sample, complexes of the antibody immobilized to the reagent immobilization part 13, the target substance, and the marker reagent are formed in accordance with the amount of the target substance.

Next, the liquid sample reaches the reagent immobilization part II4. When the target substance exists in the liquid sample, complexes of the antibody immobilized to the reagent immobilization part II4, the target substance, and the marker reagent are formed in accordance with the amount of the target substance.

Further, the liquid sample reaches the open part 7 in the developing layer 1. Since the open part 7 is opened without being covered with the liquid impermeable sheet 5, the liquid sample is volatilized or evaporated after it has reached or while reaching the open part 7. Further, the liquid sample exudes onto the open part 7, and only the liquid sample on the developing layer 1 at the open part 7 reaches up to the same height or the approximate height as the liquid sample existing on the developing layer 1 in the fine space 6. Generally, an absorption part is often provided instead of the open part. The reason is as follows. When a porous material having a higher water-holding effect or an absorption effect than that of the material of the developing layer 1 is adopted for the absorption part, the absorption part absorbs or sucks the liquid sample, thereby providing a function of making the sample pass on the developing layer 1, and a function of reducing the measurement time. The open part 7 has the same effects as those of the absorption part, and particularly the technique of using the fine space 6 or the open part 7 is suitable for a case where the liquid sample is very small in quantity such as blood obtained by piercing a fingertip.

A measurement value of the target substance in the liquid sample is obtained by measuring a signal derived from the marker reagent in the reagent immobilization part 13 and the reagent immobilization part 114.

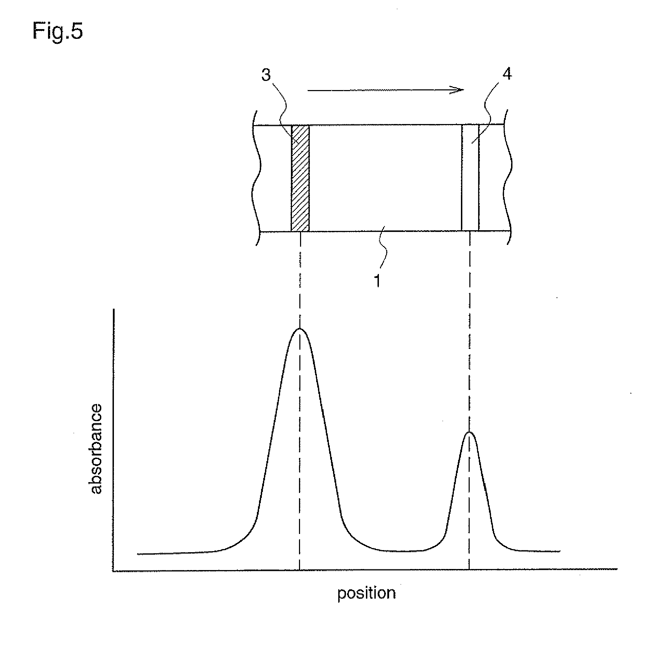

Figure 4 shows the signal measurement unit for measuring a signal derived from the marker reagent on the reagent immobilization parts 3 and 4 on the analysis element. Reference numeral 21 denotes an irradiation part for irradiating the developing layer 1 with light, and reference numeral 22 denotes a light-receiving part for detecting an optical change such as reflection or transmission of the light radiated from the irradiation part 21 to the developing layer 1. The signal measurement unit 20 measures a signal derived from the marker reagent in the reagent immobilization parts 3 and 4 by utilizing the irradiation part 21 and the light-receiving part 22. Figure 5 schematically illustrates a result of a color reaction on the reagent immobilization part 13 and the reagent immobilization part II4, which result is read as shown in figure 4. The signal rises at the reagent immobilization parts as compared with other parts, and its intensity varies according to the degree of color in the reagent immobilization parts.

The irradiation part 21 is desired to be a visible area or an approximately visible area, and an LED (Light Emitting Diode) or an LD (Laser Diode) can be selected according to need.

Then, the marker-reagent-derived signal in the reagent immobilization parts 3 and 4 which is measured by the signal measurement part 20 is arithmetically processed in the measurement value calculation unit 70 to obtain a measurement value of the target substance. The measurement value of the target substance thus obtained is a value of the target substance which is obtained from the marker-reagent-derived signal obtained in the signal measurement unit 20 by using a calibration curve. The calibration curve is a regression formula indicating a relationship between the signal obtained by the signal measurement unit 20 and the value of the target substance in the liquid sample. When measuring a liquid sample including an unknown amount of target substance, a measurement value of the target substance in the liquid sample can be calculated by substituting the signal obtained by the signal measurement unit 20 into the formula. Further, the signal measurement unit 20 may use an arbitrary means for measuring a signal, such as a means for reading an optical change, an electric change, or a magnetic change, or a means for capturing the signal as an image.

Further, while the above-mentioned reaction is a sandwich reaction utilizing an antigen-antibody reaction, a reaction system utilizing a competition reaction may be adopted by selecting a reagent.

Furthermore, when it is desired to utilize a specific reaction, measurement by a reaction other than the antigen-antibody reaction can be performed by constituting with a reagent component of a system that forms an arbitrary reaction on the analysis element 100. As for a combination of a specific substance and a specific binding substance which perform a specific reaction, there are an antigen and an antibody thereto, complementary nucleic acid sequences, an effector molecule and a receptor molecule, an enzyme and an inhibitor, an enzyme and a cofactor, an enzyme and a ground substance, a compound having a sugar change and a lectin, an antibody and an antibody thereto, a receptor molecule and an antibody thereto, a reaction system that is chemically modified to an extent that specific binding activity is not lost, and a reaction system utilizing a complex substance that is obtained by binding with another component. However, it is hard to say that the measurement value of the target substance which is obtained by the measurement value calculation unit 70 as described above has a high precision, because it is affected by the properties of the liquid sample and the analysis element 60. Accordingly, in order to obtain a highly precise analysis value of the target substance, correction of the measurement value described hereinafter is required.

Hereinafter, a description will be given of a method for obtaining an analysis value of the target substance with a measurement error of the target substance being corrected, from the measurement value of the target substance. The correction method described hereinafter is merely an example, and there will be no problem in using other methods. As a parameter indicating a degree of influence to the measurement error of the target substance, a developing speed of the liquid sample is adopted. Further, it is possible to utilize, as a parameter, another information relating to the developing state of the liquid sample, such as a developing time required for developing an arbitrary constant distance, or a developing distance in an arbitrary constant time, or a change in absorbance of a background at an arbitrary position excluding the reagent immobilization parts on the channel.

In this first embodiment, the parameter collection unit 30 for collecting the above-mentioned parameters detects the developing state of the liquid sample by detecting an optical change using the irradiation part 31 and the light-receiving part 32. An arbitrary means other than mentioned above such as a means for reading an electric change or a magnetic change or a means for capturing such change as an image may be adopted. The arrival times of the liquid sample may be simultaneously detected using plural irradiation parts and plural light-receiving parts, or the arrival times may be measured by detecting arrival of the liquid sample to the start point and then detecting successive migrations of the liquid sample to the end point by using the same irradiation part and the same light-receiving part. Further, the irradiation part 21 and the light-receiving part 22 of the signal measurement unit 20 for measuring a signal derived from the marker reagent may be used as the irradiation part 31 and the light-receiving part 32 of the parameter collecting part 30.

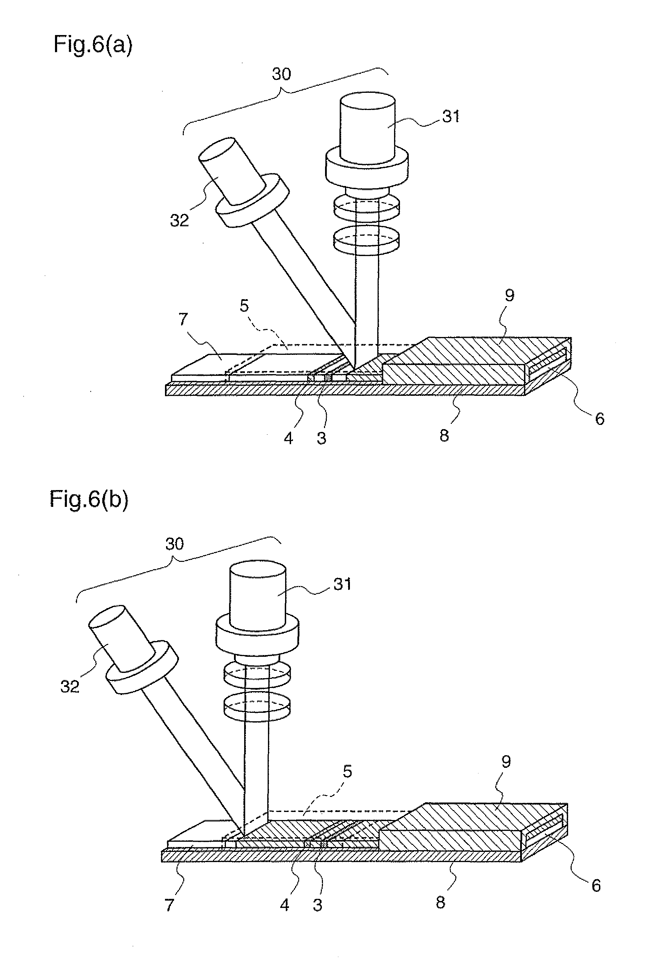

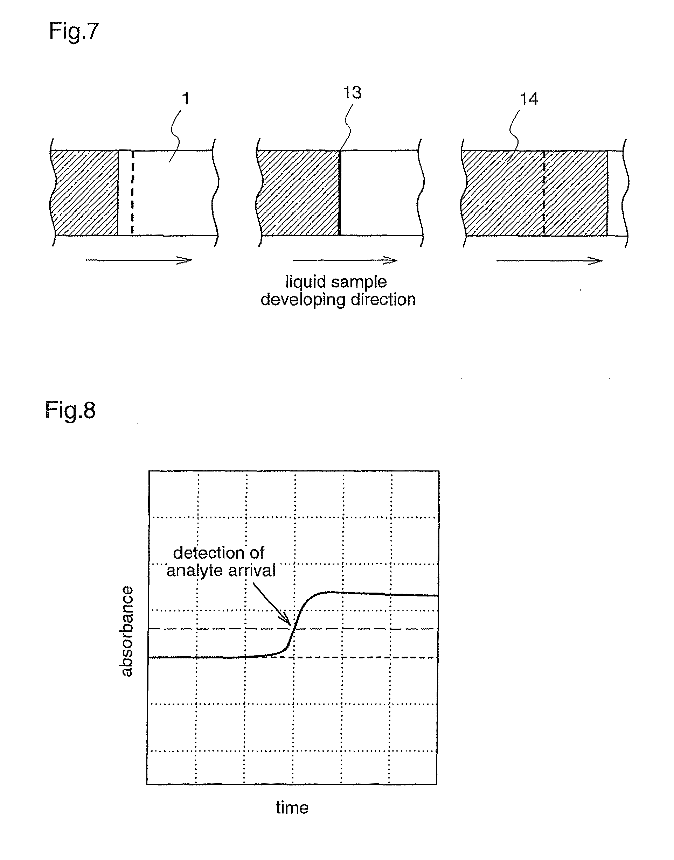

In figure 6, reference numeral 31 denotes the irradiation part for irradiating the developing layer with light, and reference numeral 32 denotes the light-receiving part for detecting an optical change such as reflection or transmission of the light emitted from the irradiation part to the developing layer. Figures 7 and 8 are diagrams before and after arrival of the liquid sample to a detection part which detects arrival of the liquid sample at a start point or an end point of an arbitrary detection section where a developing speed of the liquid sample is measured. An upstream side where the liquid sample is developed in this detection section is called a start point while a downstream side is called an end point. Figure 7 shows the developing state of the liquid sample with passage of time, and figure 8 shows an optical change in the detection part around the arrival of the liquid sample.

In figure 7, reference numeral 13 denotes the detection part, and reference numeral 14 denotes the liquid sample that is developed on the channel of the analysis element 100. As shown in figure 8, when the liquid sample 14 reaches the detection part 13, the absorbance increases. The light-receiving part detects that the liquid sample has reached the detection part when a predetermined absorbance is exceeded, and measures the arrival time of the liquid sample.

As shown in figure 6(a), initially, arrival of the liquid sample is detected from an optical change at the start point of the arbitrary detection section on the channel 1 by using the irradiation part 31 and the light-receiving part 32. The arbitrary detection section on the channel 1 is a section where the correlation between the developing speed of the liquid sample and the degree of deviation from the true value of the target substance which is the measurement error is strong, and it is selected from the part covered with the liquid impermeable sheet material. Next, as shown in figure 6(b), the irradiation part 31 and the light-receiving part 32 are scanned to the end point of the arbitrary section in the developing layer 1 as the channel on the analysis element 100, and the arrival time of the liquid sample is detected. Then, a developing time required when the liquid sample is developed in the arbitrary detection section in the developing layer 1 is calculated from these results, thereby obtaining a developing speed of the liquid sample. Further, while the developing speed calculated from the developing time in the arbitrary detection section is utilized as a parameter, the developing speed may be calculated from a developing distance in which the liquid sample is developed within an arbitrary time.

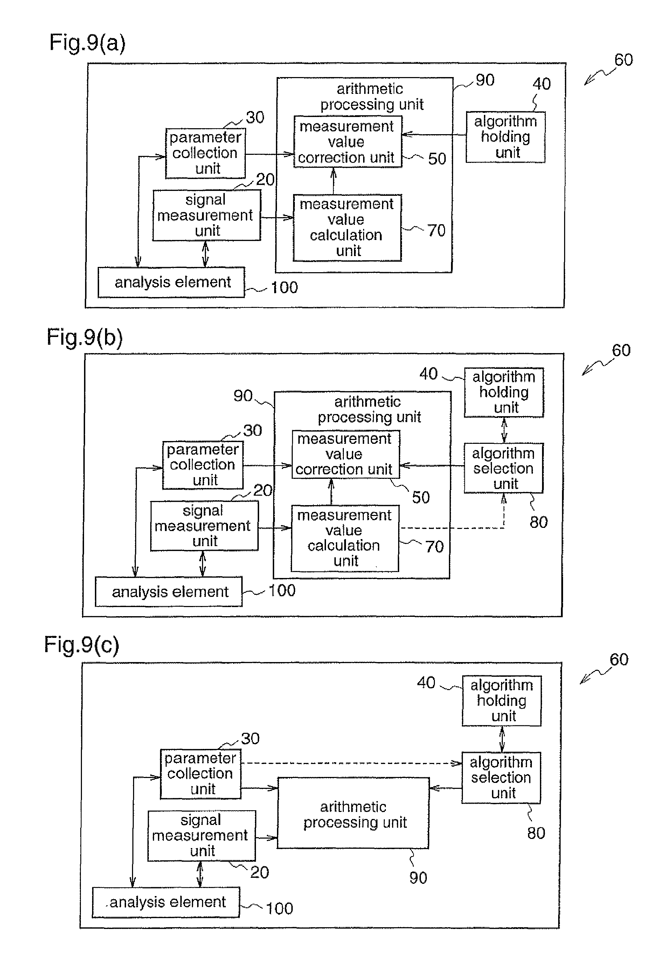

Next, the arithmetic processing unit 90 reads the algorithm from the algorithm holding unit 40, and obtains an analysis value of the target substance with the measurement error of the target substance being corrected on the basis of the parameter obtained in the parameter collection unit 30. The analysis value of the target substance is a value of the target substance which is obtained in the arithmetic processing unit 90 by correcting the measurement error of the target substance so as to minimize a difference from the true value of the target substance on the basis of the parameter obtained in the parameter collection unit 30. The algorithm is a formula for obtaining an analysis value of the target substance by correcting the measurement error of the target substance, which formula is derived from the relationship between the parameter obtained in the parameter collection unit 30 and the measurement error of the target substance, or an correction formula which is derived from the relationship between the signal obtained in the signal measurement unit 20 on the basis of the parameter and the true value of the target substance. There are various kinds of algorithm deriving methods. Although correction methods described hereinafter are preferable as simple and highly precise methods, there is no problem in adopting other methods. Figures 9(a), 9(b), and 9(c) are conceptual diagrams illustrating the following methods 1), 2), and 3), respectively.

Method 1) As shown in figure 9(a), the measurement value correction unit 50 reads the correction formula derived from the correlation between the parameter and the measurement error, which is stored in the algorithm holding unit 40, and substitutes the parameter collected by the parameter collection unit 30 and the measurement value calculated by the measurement value calculation unit 70 from the signal measured in the signal measurement unit 20 into the read correction formula, thereby correcting the measurement value of the target substance to obtain an analysis value of the target substance. For example, assuming that the measurement value of the target substance is Z and the parameter is X, the corrected analysis value Y of the target substance is obtained by the following formula.

$$Y = Z \div \{1+(aX+b)\}; (a,b:\ \text{constants})$$

The above correction formula is a numerical formula which is previously derived from the relationship between the parameter and the measurement error by using a liquid sample including an known amount of target substance and having a different degree of influence to the measurement error such as the viscosity of the liquid sample. Thereafter, when measuring a liquid sample including an unknown amount of target substance, it is possible to obtain an analysis value of the target substance by correcting the measurement value of the target substance from the obtained parameter.

Method 2) As shown in figure 9(b), plural correction formulae are prepared in the algorithm holding unit 40 according to the measurement value of the target substance that is obtained by the measurement value calculation unit 70 from the signal measured by the signal measurement unit 20, and the algorithm selection unit 80 selects any of the correction formulae according to the measurement value, and the parameter collected by the parameter collection unit 30 and the measurement value are substituted in the selected correction formula, thereby correcting the measurement value of the target substance to obtain an analysis value of the target substance. When there are, on the analysis element 100, plural parts (reagent immobilization parts) for measuring the signal based on the reaction by the signal measurement part 20, the measurement value is corrected according to each reagent immobilization part, i.e., according to the measurement value calculated from the signal obtained in each respective reagent immobilization part, whereby a more accurate analysis value can be derived.

Method 3) As shown in figure 9(c), plural calibration curves according to the parameter obtained in the parameter collection unit 30 are prepared in the algorithm holding unit 40, and the algorithm selection unit 80 selects any of the calibration curves according to the parameter, and the arithmetic processing unit 90 substitutes the signal measured in the signal measurement unit 20 into the selected calibration curve, thereby obtaining an analysis value of the target substance with the measurement error of the target substance being corrected. The calibration curves to be selected according to the parameter are derived by using liquid samples including known amounts of target substance, in which components that may influence on the measurement precision and the developing state of the liquid sample such as a hematocrit value and a total protein concentration are adjusted within a wide range that is clinically assumed. That is, in this method, the signal obtained in the signal measurement unit 20 is not input to the measurement value calculation unit 70 to detect a measurement value of the target substance, but the signal is directly substituted in the calibration curve selected based on the parameter to obtain an analysis value of the target substance with the measurement error of the target substance being corrected.

Further, as for the algorithm holding part 40 which holds the algorithm to be read out when the arithmetic processing unit 90 performs correction, it may be incorporated in the analysis device 60 as a circuit, or it may be convertibly stored by using a storage medium or the like, or it may be input to the analysis device 60 at measurement and made to perform operation after measurement to display the analysis value of the target substance. However, there is no problem in using other methods. Further, it is also possible to adopt a method of inputting a specific constant to the analysis device 60 or the analysis element 100 for a correlation formula that is previously incorporated in the analysis device 60 as a circuit, and this method is preferable in considering the lot difference of the analysis device 60 or the analysis element 100. When part or all of the constant parts of the function depends on the lot of the analysis device 60 or the analysis element 100, these constant parts are set in the device before analysis is started, thereby eliminating an influence due to the lot difference of the analysis device 60 or the analysis element 100. Further, while in figures 9(b) and 9(c) the algorithm selection unit 80 is provided outside the arithmetic processing unit 90, the algorithm selection unit 80 may be included in the arithmetic processing unit 90.

Examples of major factors of measurement errors which can be corrected by the analysis device 60 of the present invention will be described hereinafter. By performing correction with considering the measurement error factors I, it is possible to reduce deterioration in precision due to the measurement error factors II and III.

I. Properties of liquid samples
types of liquid samples
e.g., whole blood, blood plasma, blood serum, urine, bacterial cell suspending solution, etc.
characteristics of liquid samples
e.g., viscosity of the liquid sample, amount of formed element, hematocrit value when the liquid sample is whole blood, total protein concentration, etc.
additive amount of liquid sample
e.g., excess or shortage of additive amount, deficient additive amount, etc.
II. Properties of analysis element

e.g., change in activity of the reagent relating to specific binding, change in property of the material of such as the developing layer that forms a channel, imperfect development of the liquid sample due to dust or contamination on the channel

III. Analysis envelopment

e.g., temperature and humidity during measurement

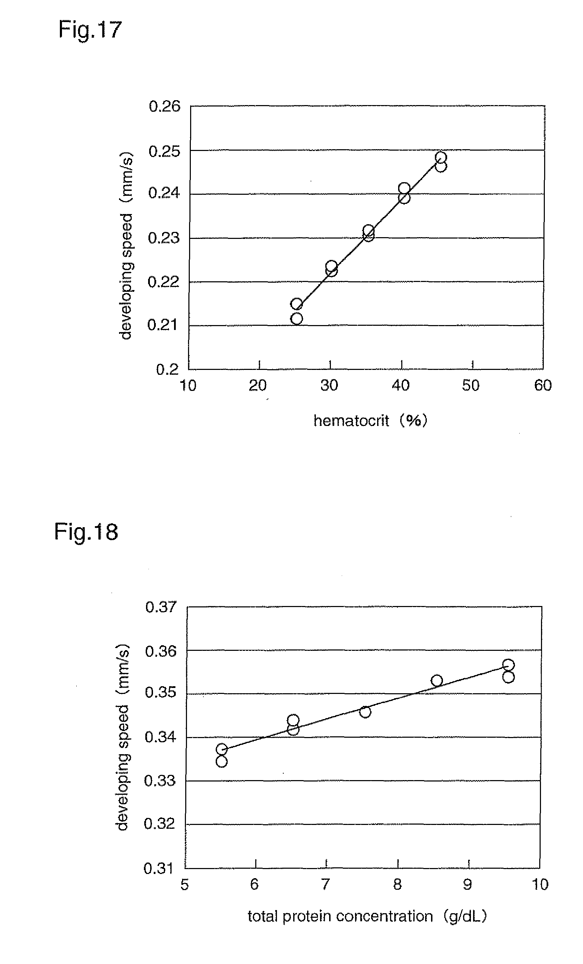

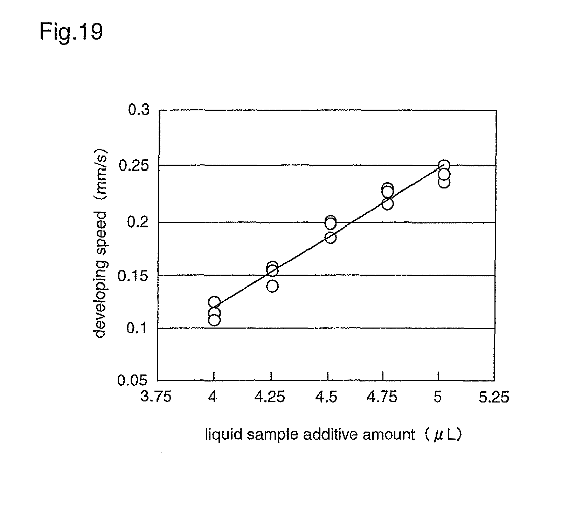

The fluidity of the liquid sample varies due to these measurement error factors, and thereby differences occur in the reaction time, which adversely affect the precision of the measurement value of the target substance. For example, as an influence on the measurement error of the liquid sample due to the property of the liquid sample, as shown in figure 17, when the liquid sample is whole blood, there is a tendency that the developing speed is lowered with an increase in the hematocrit value. Further, as shown in figure 18, when the liquid sample is blood plasma, there is a tendency that the developing speed is lowered with an increase in the total protein concentration. Further, as shown in figure 19, there is a tendency that the developing speed is lowered with a decrease in the additive amount of the liquid sample. However, these examples are merely small parts, and it is possible to correct even measurement error factors which are not clearly specified here.

As described above, according to the analysis device of the first embodiment of the present invention, when measuring a target substance in a liquid sample, a measurement error of the target substance is easily corrected according to a degree of influence on the measurement error due to the property of the liquid sample or the analysis device to obtain an analysis value of the target substance. Therefore, it is possible to provide a simple, speedy, and highly-precise analysis device.

Methods for executing the present invention will be described in more detail using the following examples. However, the present invention is not restricted to the following examples.

Example 1

(quantitative determination for whole blood CRP with an influence of hematocrit being corrected)

An immunochromatography sensor as an analysis element including a reagent immobilization part I obtained by immobilizing anti-CRP antibody A on a nitrocellulose film, a reagent immobilization part II obtained by immobilizing anti-CRP antibody B on the nitrocellulose film, and a marker reagent part holding complexes of anti-CRP antibody C and gold colloid (marker reagent) was manufactured. This immunochromatography sensor is shown in figures 2 and 3. In the figures, the immunochromatography sensor includes the reagent immobilization part 13 and the reagent immobilization part II4 on which the antibodies are immobilized, the marker reagent part 2 as an area containing the complexes of anti-CRP antibody C and gold colloid, which is closer to a part where a liquid sample is applied than the reagent immobilization parts 3 and 4, and a sample application part 6. This immunochromatography sensor was manufactured as follows.

a) Preparation for immunochromatography sensor

An anti-CRP antibody A solution whose concentration is adjusted by dilution with a phosphate buffer solution was prepared. This antibody solution was applied on a nitrocellulose film by using a solution discharge unit. Thereby, an immobilized antibody line I3 as a reagent, immobilization part was obtained on the nitrocellulose film. Next, similarly, an anti-CRP antibody B solution was applied to a portion that is apart by 2mm downstream from the sample application part. After this nitrocellulose film was dried, it was immersed in a Tris-HCl buffer solution containing 1% skim milk, and shaken gently for 30 minutes. After 30 minutes, the film was moved into a Tris-HCl buffer solution tank and shaken gently for 10 minutes, and thereafter, it was shaken gently for another 10 minutes in another Tris-HCl buffer solution tank, thereby washing the film. Next, the film was immersed in a Tris-HCl buffer solution containing 0.05% sucrose monolaurate, and shaken gently for ten minutes. Thereafter, the film was taken out of the solution tank, and dried at room temperature. Thus, the immobilized antibody line 13 and the immobilized antibody line II4 as reagent immobilization parts were obtained on the nitrocellulose film.

The gold colloid was prepared by adding a 1% citric acid solution to a 0.01% chlorauric acid 100°C solution that is circulated. After the circulation was continued for 30 minutes, the solution was cooled by being left at room temperature. Then, the anti-CRP antibody C was added to the gold colloid solution that is adjusted to pH8.9 with a 0.2M potassium carbonate solution, and the solution was shaken for several minutes. Thereafter, a 10% BSA (bovine serum albumin) solution of pH8.9 was added to the solution by such an amount that the concentration thereof finally becomes 1%, and the solution was stirred, thereby preparing antibody-gold colloid complexes (marker antibody). The marker antibody solution was subjected to centrifugation at 4°C and 20000G for 50 minutes to isolate the marker antibody, and then the marker antibody was suspended in a washing buffer solution (1% BSA 5% sucrose·phosphoric acid solution), and subjected to centrifugation to wash and isolate the marker antibody. The marker antibody was suspended in a washing buffer solution, and filtered with a 0.8μm filter, and thereafter, adjusted so that the absorbance at 520nm becomes 150, and then stored at 4°C. The marker antibody solution was set in a solution discharge device, and applied to a position apart from the immobilized line I and the immobilized line II on the dried film to which the immobilization anti-CRP antibody A and the immobilization anti-CRP antibody B are applied so as to have a positional relationship of the marker antibody, the immobilized line I, and the immobilized line II arranged in this order from the liquid sample application start position, and thereafter, the film was subjected to vacuum freeze dry. Thereby, a reaction layer carrier having the reagent immobilization parts and the marker reagent part was obtained.

Next, the reaction layer carrier including the prepared marker reagent was bonded to a substrate 8 comprising a 0.5mm thick white PET, and a transparent tape was bonded thereto from the marker reagent part 2 to the end part. Thereafter, the substrate 8 was cut into widths of 2.0mm using laser. After the cutting, a fine space formation member 9 formed by laminating 100 μm thick transparent PET was bonded onto the front end portion where the transparent tape is not bonded, thereby forming a fine space 6 (width 2.0mm×length 12.0mm×height 0.5mm). A 10% potassium chloride solution was previously applied to the space formation member 9, and then the space formation member 9 was quickly frozen by liquid nitrogen and freeze-dried, thereby forming the space formation member which holds the cell contraction agent that is potassium chloride being held in its dry state. Thus, the immunochromatography sensor was manufactured.

b) Preparation of liquid sample

CRP solutions of known concentrations were added to human blood to which heparin was added as an anticoagulant, thereby preparing blood having CRP concentrations of 0.6mg/dL and 5mg/dL. Further, the total protein concentration was set to 7.5g/dL, and the hematocrit value was adjusted to 20%, 30%, 40%, and 50%.

c) Measurement of degree of coloration on immunochromatography sensor

The whole blood containing CRP which was prepared in the step b) was applied by about 5μL to the sample application part of the immunochromatography sensor, and developed toward the open part to promote an antigen-antibody reaction. The coloration status on the antibody immobilized part after 5 minutes from the sample application to the immunochromatography sensor was measured. Figure 4 is a diagram for explaining the measurement in the first example. In figure 4, an irradiation part 21 is a light source comprising a 635nm semiconductor laser, and a light-receiving part 22 is constituted by a light-receiving element (photodiode). Further, the immunochromatography sensor side was scanned, and the amounts of the marker reagent bound onto the reagent immobilization part 13 and the reagent immobilization part II4 were obtained as absorbances by arithmetically processing the reflected and scattered light from the developing layer. Figure 5 shows a measured waveform diagram according to the first example of the present invention. In figure 5, two reagent immobilization parts are provided, and an antibody having a higher affinity was used for the reagent immobilization part on the upper stream side with respect to the sample application part. The light source and the photoreceptor were fixed, and the immunochromatography sensor side was scanned, and peak values (reflection absorbances) were read from the obtained waveform. The light source side may be scanned to obtain such waveform.

Next, the reflection absorbances obtained in the reagent immobilization part 13 and the reagent immobilization part 114 were substituted in the respective calibration curves that have been prepared, thereby obtaining the CRP concentrations.

d) Selection of detection section where parameter is collected

Hereinafter, selection of a detection section for a developing speed as a parameter will be described. Using the irradiation part 31 and the light-receiving part 32 of the parameter collection unit 30 shown in figure 6, a developing speed of the liquid sample in an arbitrary detection section on the channel and reflection absorbances in the reagent immobilization parts I and II were measured, and a relationship between the developing speed and the degree of deviation from the true value of the CRP concentration which is a measurement error was observed. The true value of the CRP concentration used for calculation of the deviation degree was measured using a commercially-available automatic analysis device (Hitachi7020 produced by Hitachi,Ltd.) to which the whole blood prepared in the step b) has previously been dispensed. Here, the developing speed of the liquid sample was used as a parameter.

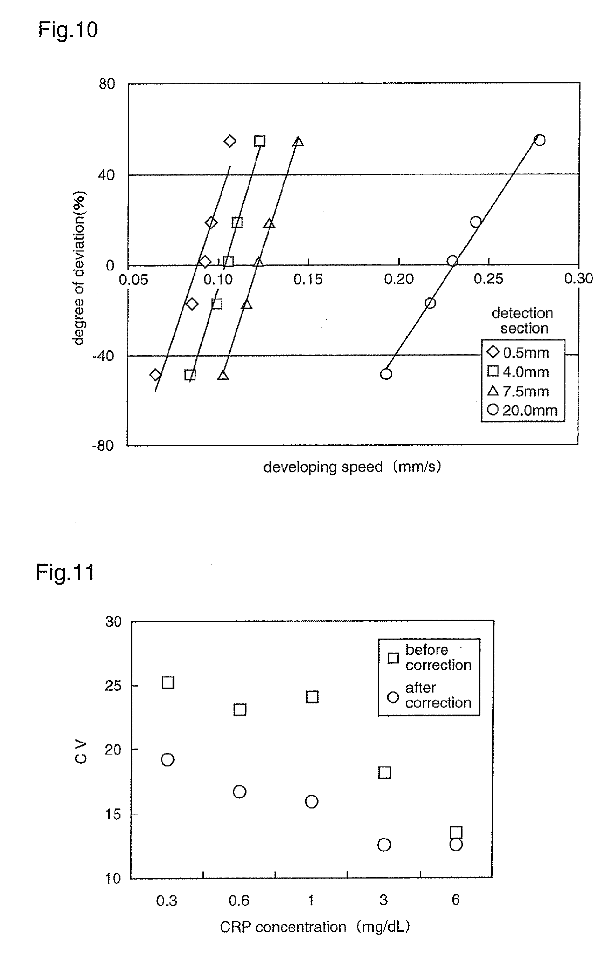

Initially, the detection section for the developing speed as a parameter is varied to 0.5, 4.0, 7.5, 20.0mm, and the developing speeds of the liquid sample in the respective distances were calculated. Next, the reflection absorbances obtained at the reagent immobilization part I and the reagent immobilization part II were substituted in the prepared calibration curves to calculate measurement values of expected CRP concentrations, and degrees of deviation from the true value of the CRP concentration of the liquid sample used in this measurement were obtained. The relationship between the developing speeds and the deviation degrees is shown in figure 10. From this result, very preferable correlations were shown when the detection section is 4.0, 7.5, and 20.0mm. When the developing speed is high, the amount of passage of the CRP as the target substance in the sample increases in the reaction part, and thereby the measurement value tends to be higher than the true value of the CRP concentration. On the other hand, when the developing speed is low, the amount of passage of the CRP decreases in the reaction part, and thereby the measurement value tends to be lower than the true value of the CRP. The measurement value indicates a CRP concentration obtained by substituting the absorbance in the prepared calibration curve.

For example, when the parameter detection section is 4.0mm, the correlation equation between the developing speed x and the deviation degree y is represented by the following formula (1).

$$y = 26.258x-2.7281 \qquad \ldots (1)$$

Based on this correlation equation, a numerical formula for correcting the CRP concentration from the developing speed can be derived. Assuming that the measurement value of the CRP concentration is Z, a correction formula for obtaining an analysis value Y of the CRP concentration is represented by the following formula (2).

$$Y = Z \div \{1+(26.258x-2.7281)\} \qquad \ldots (2)$$

e) Derivation of mathematical formula for correcting influence of hematocrit value

Here, an area of 20.0mm from the start end to the middle of the channel in which the correlation between the developing speed and the deviation degree was largest in the inspection of the detection section in step d) was adopted as a detection section, and a correction formula that is derived from the correlation between the developing speed of the liquid sample in this detection section and the degree of deviation from the true value of the CRP concentration was used. The correlation formula was used separately for the case where the measurement value is less than 1.0mg/dL and the case where it is equal to or larger than 1.0mg/dL. Assuming that the measurement value of the CRP concentration is Z and the developing speed is x, correction formulae for obtaining analysis values Y of the CRP concentration were represented by the following formulae 3 and 4.

When the measurement value is less than 1.0mg/dL;

$$Y = Z \div \{1+(6.3589x-1.3949)\} \qquad \ldots (3)$$

When the measurement value is equal to or larger than 1.0mg/dL;

$$Y = Z \div \{1+(3.8233x-0.61879)\} \qquad \ldots (4)$$

f) Correction of influence due to hematocrit value

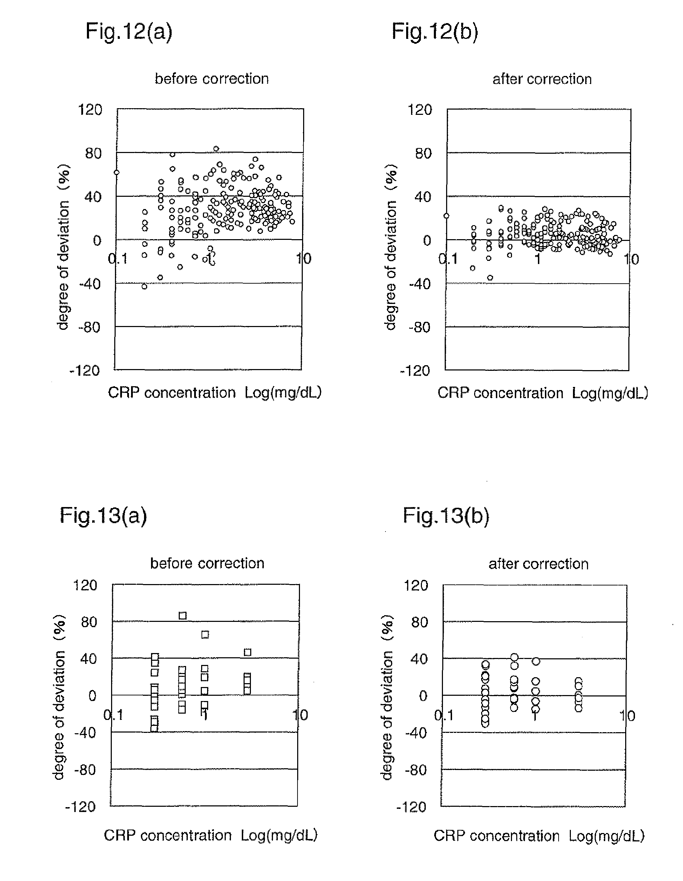

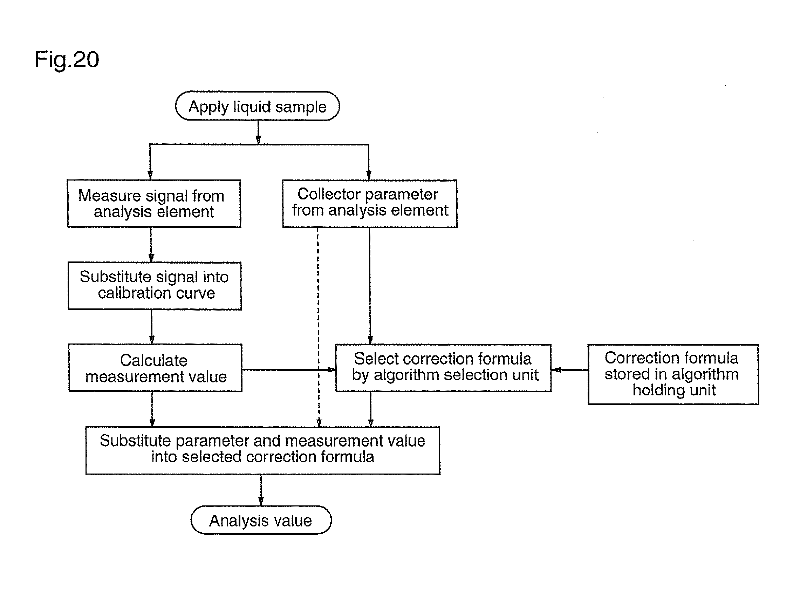

The algorithm selection unit 80 selects any of the correction formulae stored in the algorithm holding unit 40 according to the measurement value of the CRP concentration, and the measurement value correction unit 50 substitutes the parameter and the measurement value into the selected correction formula, whereby the measurement value of the CRP concentration was corrected and an analysis value of the CRP concentration was obtained. A flowchart thereof is shown in figure 20. Figure 11 shows the CV values before and after the correction. The CV value before the correction reflects the influence of the hematocrit value, and the CV value must be improved to obtain a more reliable analysis value of the CRP concentration. In contrast, it is evident from figure 11 that the measurement precision is significantly improved when correction of the measurement value is performed based on the parameter derived from the developing speed of the liquid sample. Further, figure 12 shows distribution of deviation degrees before and after the correction. The abscissa shows the true value of the CRP concentration, and the ordinate shows the degree of deviation. Before the correction, the deviation was significantly large when the hematocrit value was low. In contrast, after the correction has been performed, the deviation degree decreases dramatically, and a sufficient correction effect was obtained. It seems that more accurate measurement is possible by using this correction method.

Example 2

(quantitative determination for whole blood CRP with influence of total protein concentration being corrected)

An immunochromatography sensor as an analysis element including a reagent immobilization part I obtained by immobilizing anti-CRP antibody A on a nitrocellulose film, a reagent immobilization part II obtained by immobilizing anti-CRP antibody B on the nitrocellulose film, and a marker reagent part holding complexes of anti-CRP antibody C and gold colloid (marker reagent) was manufactured. This immunochromatography sensor is shown in figures 2 and 3. This immunochromatography sensor was manufactured as follows.

a) Preparation for immunochromatography sensor

The following measurement was performed using a sensor in the same lot as the immunochromatography sensor used in the first example.

b) Preparation of liquid sample

CRP solutions of known concentrations were added to human blood to which heparin was added as an anticoagulant, thereby preparing blood having CRP concentrations of 0.6mg/dL and 5mg/dL. Further, the hematocrit value was set to 40%, and the total protein concentration was adjusted to 2.5g/dL, 5g/dL, 7.5g/dL, 10g/dL, and 12.5g/dL.

c) Measurement of degree of coloration on immunochromatography sensor

The whole blood containing CRP which was prepared in the step b) was applied by about 5μL to the sample application part of the immunochromatography sensor, and measurement values of the CRP concentrations were calculated by the same method as described for the first example.

d) Correction of influence due to total protein concentration utilizing formula for correcting influence due to hematocrit value

The measurement values of the CRP concentrations were corrected on the basis of the correction formula derived in the above-described first example to obtain an analysis value of the CRP concentration. Figure 13 shows the measurement results before and after the correction. In figure 13, the abscissa shows the true value of the CRP concentration, and the ordinate shows the degree of deviation. Before the correction, deviation was considerably large influenced by the total protein concentration. In contrast, when the correction was performed using the above-described correction formula, a correction effect against the factor of the total protein concentration difference between the liquid samples was clearly recognized. In this way, when the liquid sample is whole blood, it is possible to correct the influence due to the total protein concentration by using the same correction formula as that used for correcting the influence of the hematocrit value.
By using this correction formula, in a blood sample having a hematocrit value of 14.9~51%, a total protein concentration of 4.2~10g/dL, and an albumin concentration of 1.4~4.9%, the measurement precision was significantly improved, and a correction effect against the influences of the properties of the blood sample was clearly recognized.

Example 3

(quantitative determination for whole blood CRP with influence of shortage in additive amount of liquid sample being corrected)

An immunochromatography sensor as an analysis element including a reagent immobilization part I obtained by immobilizing anti-CRP antibody A on a nitrocellulose film, a reagent immobilization part II obtained by immobilizing anti-CRP antibody B on the nitrocellulose film, and a marker reagent part holding complexes of anti-CRP antibody C and gold colloid (marker reagent) was manufactured. This immunochromatography sensor is shown in figures 2 and 3. This immunochromatography sensor was manufactured as follows.

a) Preparation for immunochromatography sensor

The following measurement was performed using a sensor in the same lot as the immunochromatography sensor used in the first example.

b) Preparation of liquid sample

A CRP solution of a known concentration was added to human blood to which heparin was added as an anticoagulant, thereby preparing blood having a CRP concentration of 5mg/dL. Further, the total protein concentration was set at 7.5g/dL, and the hematocrit value was adjusted to 30%, 40%, and 50%.

c) Measurement of degree of coloration on immunochromatography sensor

The whole blood including CRP which was prepared in the step b) was applied to the sample application part of the immunochromatography sensor by amounts of 4μL, 4.25μL, 4.5μL, and 4.75μL which are shorter than the standard additive amount of the liquid sample and by an amount of about 5μL which is the standard additive value of the liquid sample, and measurement values of CRP concentrations were calculated by the same method as adopted in the first example.

d) Correction of influence due to shortage in additive amount of liquid sample utilizing the formula for correcting influence of hematocrit value

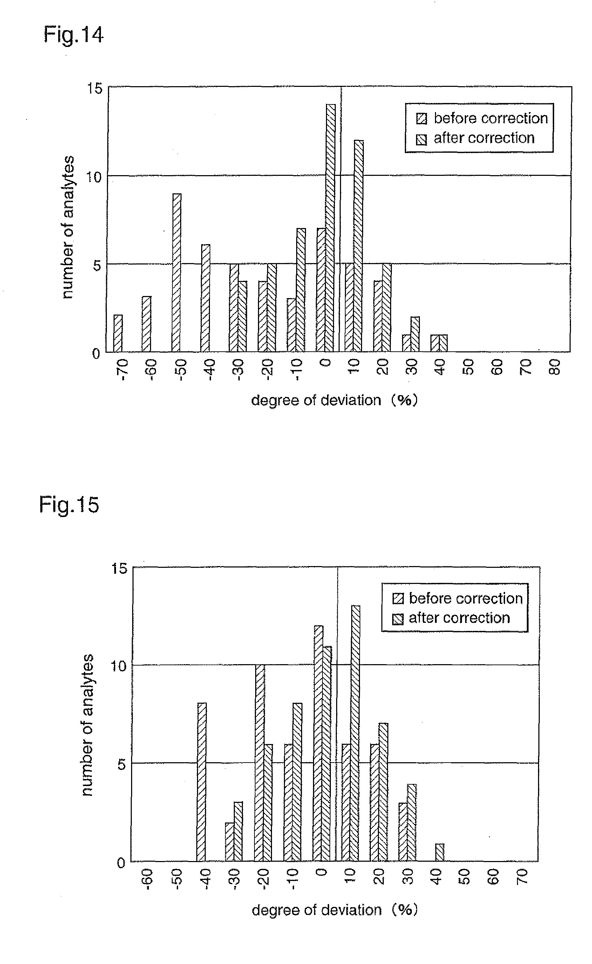

The measurement values of the CRP concentrations were corrected on the basis of the correction formula derived in the above-described first example to obtain an analysis value of the CRP concentration. Figure 14 shows distribution of deviation degrees of the measurement results before and after the correction. In figure 14, the abscissa shows the deviation degree, and the ordinate shows the number of analytes. Before the correction, the measurement results deviated to lower values influenced by the shortage in the additive amount of the liquid sample. In contrast, when the correction was performed using the above-described correction formula, a correction effect against the factor of the shortage in the liquid sample additive amount was clearly recognized.

By using this correction formula, the precision was significantly improved in the measurement in which the measurement results deviated to lower values due to shortage in the additive amount of the liquid sample, and a correction effect against the influence of the shortage in the additive amount of the liquid sample was clearly recognized. By using this correction method, it is possible to avoid a reduction in sensitivity due to shortage in the additive amount.

Example 4

(quantitative determination for whole blood CRP with influences of hematocrit and total protein concentration being corrected)

An immunochromatography sensor as an analysis element including a reagent immobilization part I obtained by immobilizing anti-CRP antibody A on a nitrocellulose film, a reagent immobilization part II obtained by immobilizing anti-CRP antibody B on the nitrocellulose film, and a marker reagent part holding complexes of anti-CRP antibody C and gold colloid (marker reagent) was manufactured. This immunochromatography sensor is shown in figures 2 and 3. This immunochromatography sensor was manufactured as follows.

a) Preparation for immunochromatography sensor

The following measurement was performed using a sensor in the same lot as the immunochromatography sensor used in the first example.

b) Preparation of sample

CRP solutions of known concentrations were added to human blood to which heparin was added as an anticoagulant, thereby preparing blood having CRP concentrations of 0.6mg/dL and 5mg/dL. Further, the hematocrit value and the total protein concentration were set to 20% 4g/dL, 30% · 5.5g/dL, 40% · 7g/dL, 50% · 8.5g/dL, 60% · 10g/dL, 30% · 8.5g/dL, and 50% · 5.5g/dL.

c) Measurement of degree of coloration on immunochromatography sensor

The whole blood containing CRP which was prepared in the step b) was applied by about 5μL to the sample application part of the immunochromatography sensor, and measurement values of the CRP concentrations were calculated by the same method as described for the first example.

d) Derivation of numerical formula for correcting influences due to hematocrit value and total protein concentration

The developing time of the liquid sample in an arbitrary section on the developing layer and the absorbances at the reagent immobilization parts I3 and II4 were measured using the irradiation part 31 and the light-receiving part 32 of the parameter collection unit 30 shown in figure 6, and the relationship between the developing time and the degree of deviation from the true value of the CRP concentration was observed. The true value of the CRP concentration used for calculation of the deviation degree was measured by using a commercially-available automatic analysis device (Hitachi7020 produced by Hitachi,Ltd.) to which the whole blood prepared in the step b) has previously been dispensed. Initially, the developing speed of the liquid sample was calculated. Next, the absorbances at the reagent immobilization parts 13 and II4 were substituted in calibration curves that had previously been formed to calculate expected CRP concentrations, and the degrees of deviation from the true value of the CRP concentration of the blood sample used in this measurement were obtained. Figure 10 shows the relationship between the developing speed and the deviation degree. When the developing speed is high, the amount of passage of the CRP as the target substance in the sample increases in the reaction part, and thereby the measurement value tends to be higher than the true value of the CRP concentration. On the other hand, when the developing speed is low, the amount of passage of the CRP as the target substance decreases in the reaction part, and thereby the measurement value tends to be lower than the true value of the CRP.

Based on the correlation equation between the developing speed and the deviation degree, a numerical formula for correcting the measurement value of the CRP concentration from the developing speed was derived. Assuming that the measurement value of the CRP concentration is Z and the developing speed is x, a correction formula for obtaining an analysis value Y of the CRP concentration is represented by the following formula (5).

$$Y = Z \div \{1 + (1.2825 Ln(x) - 2.57000)\} \qquad \ldots (5)$$

e) Correction of influences due to hematocrit value and total protein concentration

The measurement value of the CRP concentration was corrected by the measurement value correction unit 50 to obtain an analysis value of the CRP concentration. The result was shown in figure 15. Figure 15 is a diagram showing distribution of the deviation degrees of the measurement results before and after the correction, and the abscissa shows the deviation degree while the ordinate shows the number of analytes. Before the correction, the measurement results largely deviated influenced by the individual difference in the property of the liquid sample. In contrast, after the influences due to the hematocrit value and the total protein concentration were corrected, a correction effect was clearly recognized. It seems that more accurate measurement is possible by using this correction method.

Example 5

(quantitative determination for whole blood CRP corrected by plural calibration curves based on parameters)

An immunochromatography sensor as an analysis element including a reagent immobilization part I obtained by immobilizing anti-CRP antibody A on a nitrocellulose film, a reagent immobilization part II obtained by immobilizing anti-CRP antibody B on the nitrocellulose film, and a marker reagent part holding complexes of anti-CRP antibody C and gold colloid (marker reagent) was manufactured. This immunochromatography sensor is shown in figures 2 and 3. This immunochromatography sensor was manufactured as follows.

a) Preparation for immunochromatography sensor

The following measurement was performed using a sensor in the same lot as the immunochromatography sensor used in the first example.

b) Preparation of sample

CRP solutions of known concentrations were added to human blood to which heparin was added as an anticoagulant, thereby preparing blood having CRP concentrations of 0.6mg/dL and 5mg/dL. Further, the hematocrit value and the total protein concentration were adjusted to 20% · 4g/dL, 30% · 5.5g/dL, 40% · 7g/dL, 50% · 8.5g.dL, 60% · 10g/dL, 30% · 8.5g/dL, and 50% · 5.5g/dL.

c) Measurement of degree of coloration on immunochromatography sensor

The whole blood containing CRP which was prepared in the step b) was applied by about 5μL to the sample application part of the immunochromatography sensor, and reflection absorbances at the reagent immobilization parts 3 and 4 were measured by the signal measurement unit 20.

d) Derivation of numerical formulae for correcting influences of hematocrit value and total protein concentration

The developing time of the liquid sample in an arbitrary section on the developing layer and the reflection absorbances at the reagent immobilization parts I3 and II4 were measured using the irradiation part 31 and the light-receiving part 32 of the parameter collection unit 30 shown in figure 6, and the relationship between the reflection absorbance and the true value of the CRP concentration was observed for each predetermined developing speed. The true value of the CRP concentration was measured by using a commercially-available automatic analysis device (Hitachi 7020 produced by Hitachi,Ltd.) to which the whole blood prepared in the step b) was dispensed. Plural calibration curves were prepared for every predetermined developing speed according to the relationship between the reflection absorbance and the CRP concentration. Assuming that the reflection absorbance is z, the calibration curves of the reagent immobilization part I for obtaining an analysis value Y of the CRP concentration are represented by the following formulae 6 to 10 for the respective developing speeds.

When the developing speed is less than 0.100mm/s;

$$Y = 10\{(Log(z)-0.1363)/-0.5663\} \qquad \ldots(6)$$

When the developing speed is equal to or larger than 0.100mm/s and less than 0.110mm/s;

$$Y = 10\{(Log(z)-0.2642)/-0.4162\} \qquad \ldots(7)$$

When the developing speed is equal to or larger than 0.110mm/s and less than 0.120mm/s;

$$Y = 10\{(Log(z)-0.3185)/-0.4628\} \qquad \ldots(8)$$

When the developing speed is equal to or larger than 0.120mm/s and less than 0.130mm/s;

$$Y = 10\{(Log(z)-0.3661)/-0.3937\} \qquad \ldots(9)$$

When the developing speed is equal to or larger than 0.130mm/s;

$$Y = 10\{(Log(z)-0.4168)/-0.3233\} \qquad \ldots(10)$$

e) Correction of influences due to hematocrit value and total protein concentration

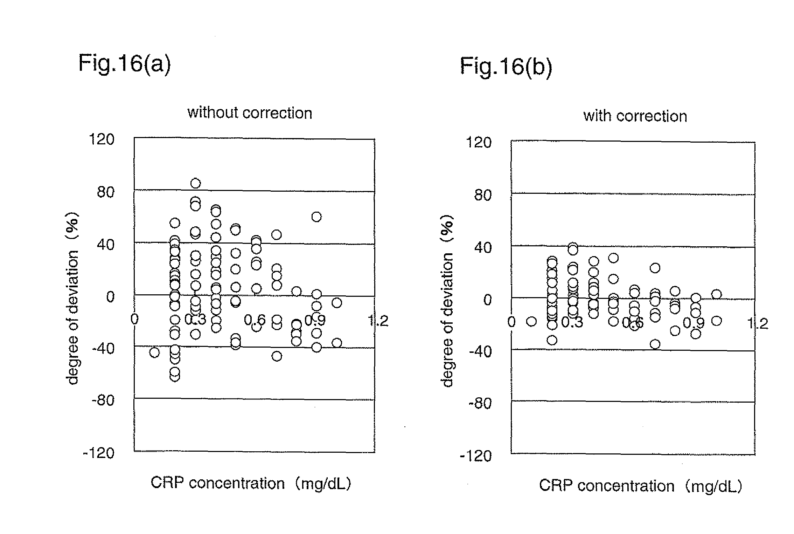

Any of the plural calibration curves stored in the algorithm holding unit 40 was selected according to the developing speed by the algorithm selection unit 80, and the signal (reflection absorbance) obtained by the signal measurement unit 20 was substituted in the selected calibration curve by the arithmetic processing unit 90, thereby obtaining an analysis value of the CRP concentration. The result is shown in figure 16. Figure 16 shows distribution of the deviation degrees of the measurement results obtained with correction and without correction. The abscissa shows the true value of the CRP concentration, and the ordinate shows the degree of deviation from the true value of the CRP concentration. When no correction was performed, the measurement results greatly deviated influenced by the individual difference in the property of the liquid sample. On the other hand, when correction was performed using the plural calibration curves based on the parameter, a correction effect was clearly recognized. It seems that more accurate measurement is possible by using this correction method.

Example 6

(quantitative determination for hCG with influences due to kinds of liquid samples being corrected)

An immunochromatography sensor as an analysis element including a reagent immobilization part I obtained by immobilizing anti-hCG antibody A on a nitrocellulose film, a reagent immobilization part II obtained by immobilizing anti-hCG antibody B on the nitrocellulose film, and a marker reagent part holding complexes of anti-hCG antibody C and gold colloid was manufactured. This immunochromatography sensor is shown in figures 2 and 3. In figures 2 and 3, the immunochromatography sensor includes the reagent immobilization part 13 and the reagent immobilization part II4 on which the antibodies are immobilized, the marker reagent part 2 as an area containing complexes of anti-hCG antibody C and gold colloid, which is closer to a part where the liquid sample is applied than the reagent immobilization parts, and the sample application part 6. This immunochromatography sensor was manufactured as follows.

a) Preparation for immunochromatography sensor

An anti-hCG antibody A solution whose concentration was adjusted by dilution with a phosphate buffer solution was prepared. This antibody solution was applied onto a nitrocellulose film by using a solution discharge unit. Thereby, an immobilized antibody line 13 as a reagent immobilization part was obtained on the nitrocellulose film. Next, similarly, an anti-hCG antibody B solution was applied to a part that is apart by 2mm to the lower stream side from the sample application part. After this nitrocellulose film was dried, it was immersed in a Tris-HCl buffer solution containing 1% skim milk, and shaken gently for 30 minutes. After 30 minutes, the film was moved into a Tris-HCl buffer solution tank and shaken gently for 10 minutes, and thereafter, it was shaken gently for another 10 minutes in another Tris-HCl buffer solution tank, thereby washing the film. Next, the film was immersed in a Tris-HCl buffer solution containing 0.05% sucrose monolaurate, and shaken gently for ten minutes. Thereafter, the film was taken out of the solution tank, and dried at room temperature. Thereby, an immobilized antibody line 13 and an immobilized antibody line II4 as reagent immobilization parts were obtained on the nitrocellulose film.

The gold colloid was prepared by adding a 1% citric acid solution to a 0.01% chlorauric acid 100°C solution that is circulated. After the circulation was continued for 30 minutes, the solution was cooled by being left at room temperature. Then, the anti-hCG antibody C was added to the gold colloid solution that is adjusted to pH8.9 with a 0.2M potassium carbonate solution, and the solution was shaken for several minutes. Thereafter, a 10% BSA (bovine serum albumin) solution of pH8.9 was added to the solution by such an amount that the concentration thereof finally becomes 1%, and the solution was stirred, thereby preparing antibody-gold colloid complexes (marker antibody). The marker antibody solution was subjected to centrifugation at 4°C and 20000G for 50 minutes to isolate the marker antibody, and then the marker antibody was suspended in a washing buffer solution (1% BSA 5% sucrose · phosphoric acid solution), and subjected to centrifugation to wash and isolate the marker antibody. The marker antibody was suspended in a washing buffer solution, and filtered with a 0.8 $\mu$m filter, and thereafter, adjusted so that the absorbance at 520nm becomes 150, and then stored at 4°C. The marker antibody solution was set in a solution discharge device, and applied to a position apart from the immobilized line I and the immobilized line II on the dried film to which the immobilization anti-hCG antibody A and the immobilization anti-hCG antibody B are applied so as to have a positional relationship of the marker antibody, the immobilized line I, and the immobilized line II arranged in this order from the liquid sample application start position, and thereafter, the film was subjected to vacuum freeze dry. Thereby, a reaction layer carrier having the reagent immobilization parts and the marker reagent part was obtained.

Next, the reaction layer carrier including the prepared marker reagent was bonded to a substrate 8 comprising a 0.5mm thick white PET, and a transparent tape was bonded thereto from the marker reagent part 2 to the end part. Thereafter, the substrate 8 was cut into widths of 2.0mm using laser. After the cutting, a fine space formation member 9 formed by laminating 100 $\mu$m thick transparent PET was bonded onto the front end portion where the transparent tape is not bonded, thereby forming a fine space 6 (width 2.0mmXlength 7.0mmXheight 0.3mm). A 10% potassium chloride solution was previously applied to the space formation member 9, and then the space formation member 9 was quickly frozen by liquid nitrogen and freeze-dried, thereby forming the space formation member which holds the cell contraction agent that is potassium chloride being held in its dry state. Thus, the immunochromatography sensor was manufactured.

b) Preparation of sample

hCG solutions of known concentrations were added to human blood to which heparin was added as an anticoagulant, thereby preparing blood having hCG concentrations of 100U/L, 1000U/L, and 10000U/L. The total protein concentration in this blood was set to 7.5g/dL, and the hematocrit value was adjusted to 20%, 30%, 40%, and 50%.

Further, hCG solutions of known concentrations were added to human blood plasma to prepare blood plasma having hCG concentrations of 100U/L, 1000U/L, and 10000U/L. The total protein concentration was set to 2.5g/dL, 5g/dL, 7.5g/dL, 10g/dL, and 12.5g/dL.

Furthermore, hCG solutions of known concentrations were added to human urine to prepare urine having hCG concentrations of 100U/L, 1000U/L, and 10000U/L.

Thus, the respective liquid sample solutions were prepared.

c) Measurement of degree of coloration on immunochromatography sensor

The whole blood containing hCG which was prepared in the step b) was applied by about 5$\mu$L to the sample application part of the immunochromatography sensor, and reflection absorbances at the reagent immobilization parts 3 and 4 were measured by the signal measurement unit 20.

d) Derivation of algorithm for identifying the kind of liquid sample

An area of 20.0mm from the start end to the middle of the channel in which the correlation between the developing speed and the deviation degree was largest in the inspection of the detection section in step d) of the first example was adopted as a detection section, and a liquid sample identification algorithm was derived from the relationship of the developing speeds that vary depending on the respective liquid samples in this detection section.

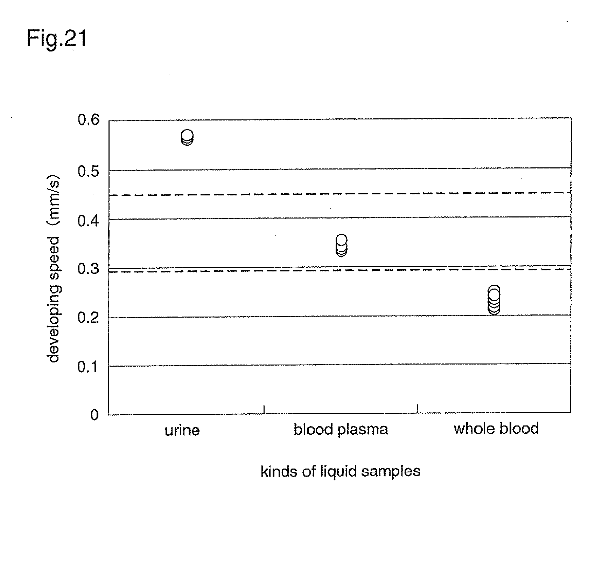

The developing speeds of the respective liquid samples are shown in figure 21. As is evident from this figure, urine, blood plasma, and whole blood have completely different developing speeds. From figure 21, a liquid sample identification algorithm which identifies the liquid sample as urine when the developing speed in the detection section is equal to or higher than 0.45mm/s, as blood plasma when it is within a range from 0.29 to 0.45mm/s, and as whole blood when it is less than 0.29mm/s, was derived.

e) Measurement of hCG concentration in urine when the liquid sample is identified as urine

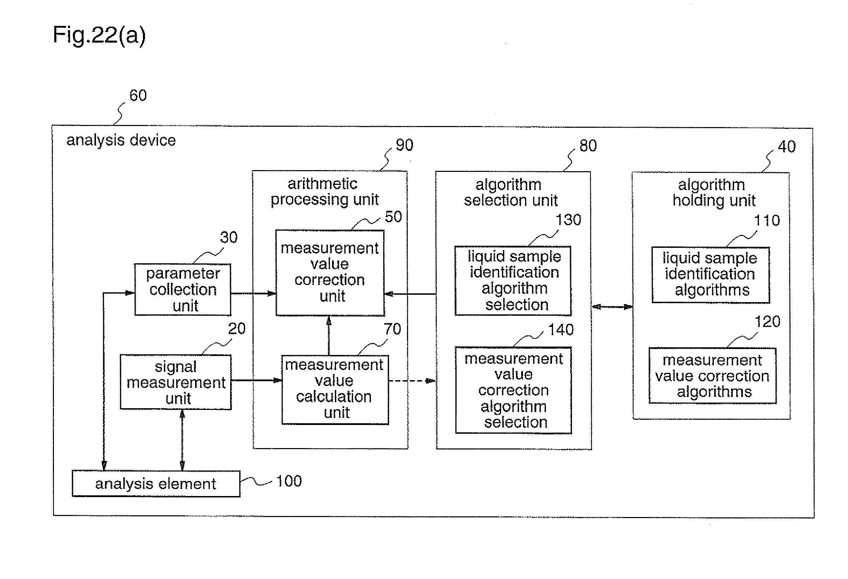

Measurement of the hCG concentration in urine will be described with reference to figure 22(a).

Initially, the liquid sample identification algorithm 110 stored in the algorithm holding unit 40 was selected by the algorithm selection unit 80, and the liquid sample was identified as urine according to the liquid sample identification algorithm. Next, the calibration curve for urine stored in the algorithm holding unit 40 was selected by the algorithm selection unit 80, and the hCG concentration in urine was calculated by the arithmetic processing unit 90.

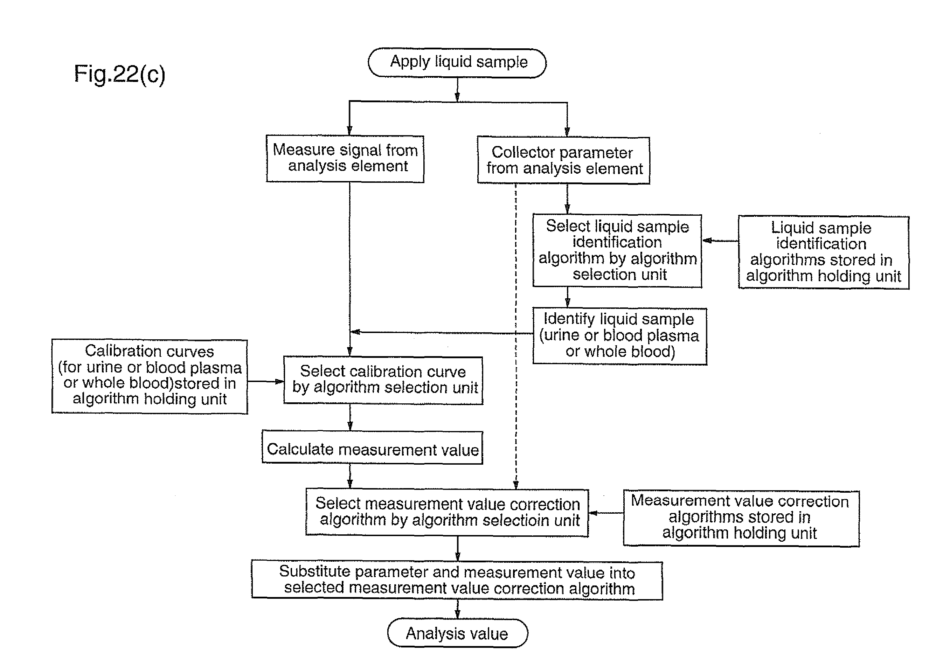

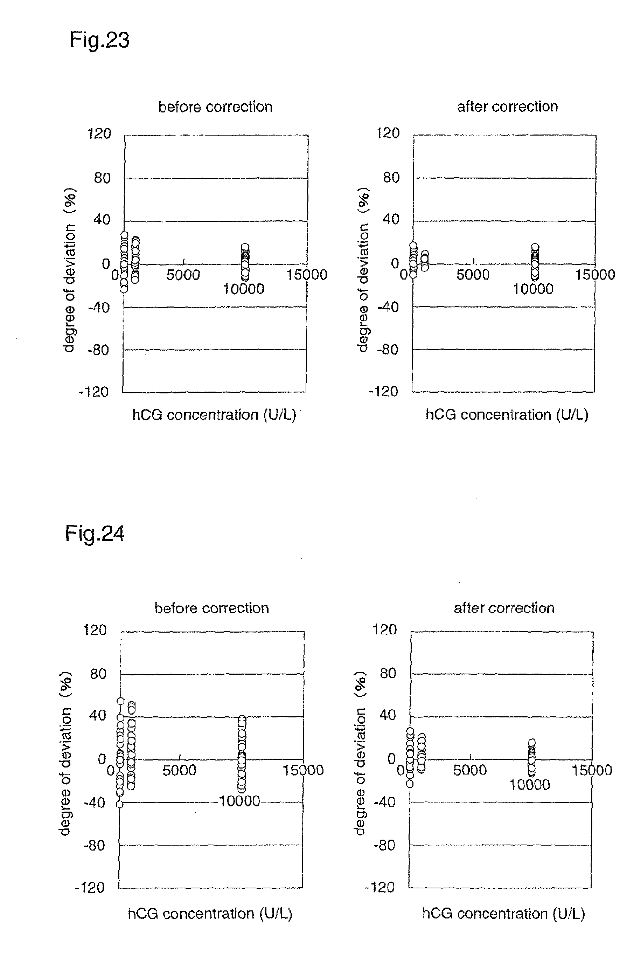

Next, the algorithm selection unit 80 selects any of the correction formulae stored in the algorithm holding unit 40 on the basis of the measurement value of the hCG concentration, and the parameter and the measurement value were substituted in the selected correction formula by the measurement value correction unit 50, whereby the measurement value of the hCG concentration was corrected to obtain an analysis value of the hCG concentration. A flowchart thereof is shown in figure 22(c). Figure 23 shows distributions of deviation degrees before and after the correction. The abscissa shows the true value of the hCG concentration, and the ordinate shows the deviation degree. Before the correction, the measurement values largely deviated when the developing speed of urine varied. In contrast, after the correction was performed, the deviation degree was remarkably reduced, and a sufficient correction effect was shown. It seems that more accurate measurement is possible by using this correction method.

f) Correction of influence due to total protein concentration when the liquid sample is identified as blood plasma

Measurement of the blood plasma hCG concentration will be described with reference to figure 22(a).

Initially, the liquid sample identification algorithm 110 stored in the algorithm holding unit 40 was selected by the algorithm selection unit 80, and the liquid sample was identified as blood plasma according to the liquid sample identification algorithm. Next, the calibration curve for blood plasma stored in the algorithm holding unit 40 was selected by the algorithm selection unit 80, and the hCG concentration in blood plasma was calculated by the arithmetic processing unit 90.

Next, the algorithm selection unit 80 selects any of the correction formulae stored in the algorithm holding unit 40 on the basis of the measurement value of the hCG concentration, and the parameter and the measurement value were substituted in the selected correction formula by the measurement value correction unit 50, whereby the measurement value of the hCG concentration was corrected to obtain an analysis value of the hCG concentration. A flowchart thereof is shown in figure 22(c). Figure 24 shows distributions of deviation degrees before and after the correction. The abscissa shows the true value of the hCG concentration, and the ordinate shows the deviation degree. Before the correction, the measurement values largely deviated when the total protein concentration was low or high. In contrast, after the correction was performed, the deviation degree was remarkably reduced, and a sufficient correction effect was shown. It seems that more accurate measurement is possible by using this correction method.

g) Correction of influence due to hematocrit value when the liquid sample is identified as blood

Measurement of the blood plasma hCG concentration will be described with reference to figure 22(a).

Initially, the liquid sample identification algorithm 110 stored in the algorithm holding unit 40 was selected by the algorithm selection unit 80, and the liquid sample was identified as whole blood according to the liquid sample identification algorithm. Next, the calibration curve for whole blood stored in the algorithm holding unit 40 was selected by the algorithm selection unit 80, and the hCG concentration in blood was calculated by the arithmetic processing unit 90.

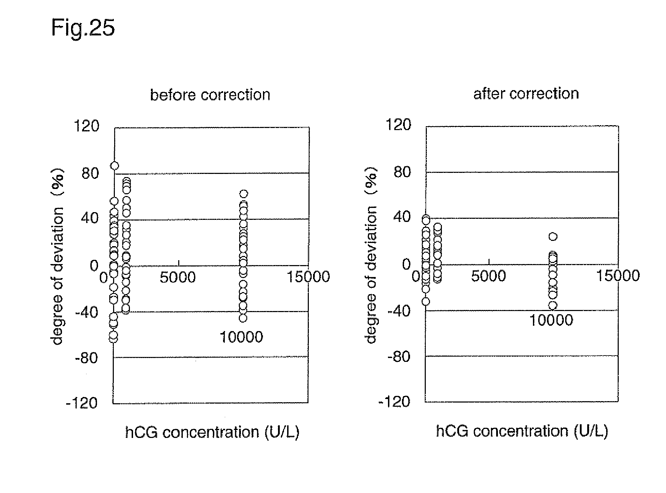

Next, the algorithm selection unit 80 selects any of the correction formulae stored in the algorithm holding unit 40 on the basis of the measurement value of the hCG concentration, and the parameter and the measurement value were substituted in the selected correction formula by the measurement value correction unit 50, whereby the measurement value of the hCG concentration was corrected to obtain an analysis value of the hCG concentration. A flowchart thereof is shown in figure 22(c). Figure 25 shows distributions of deviation degrees before and after the correction. The abscissa shows the true value of the hCG concentration, and the ordinate shows the deviation degree. Before the correction, the measurement values largely deviated when the hematocrit was low. In contrast, after the correction was performed, the deviation degree was remarkably reduced, and a sufficient correction effect was shown. It seems that more accurate measurement is possible by using this correction method.

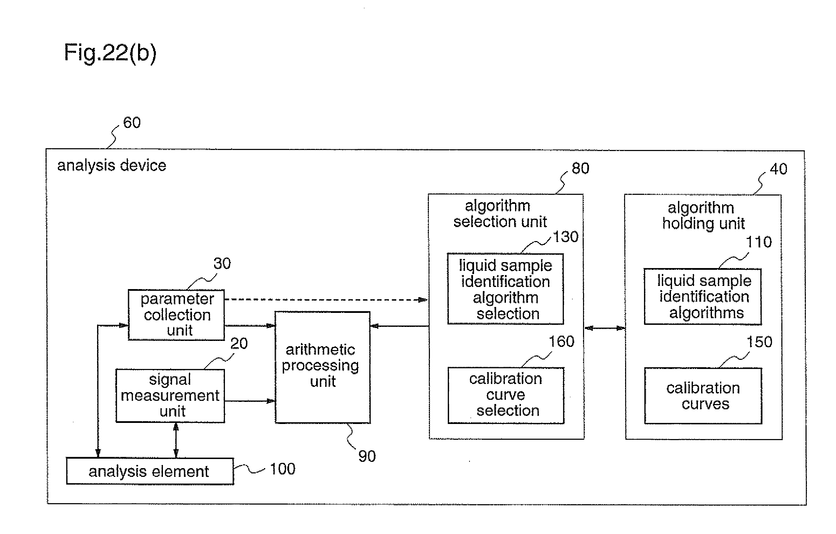

The conceptual diagrams shown in figure 22(a) and 22(b) and the flowchart shown in figure 22(c) are merely examples, and there is no problem in adopting other conceptual diagrams and flowcharts.

As described above, when the sixth example is used, quantitative measurement using a chromatography specimen can be performed regardless of the kind of the liquid sample, and the calibration curves and the correction formulae according to the characteristics of the respective liquid samples are provided. Therefore, even when any liquid sample is used, the liquid sample is automatically identified, thereby realizing highly precise quantitative measurement.

While in the first to fifth examples a sensor having a marker reagent part and sample immobilization parts provided on the same nitrocellulose film is used, a porous substrate comprising a material different from nitrocellulose such as a nonwoven fabric, on which a marker reagent is disposed, may be provided on a support member. While gold colloid is used as a marker substance constituting a marker reagent, any material may be used so long as some change occurs around a reaction, for example, coloring material, fluorescent material, phosphorescent material, light-emitting material, oxidation-reduction material, enzyme, nucleic acid, or endoplasmic reticulum may be adopted.

Furthermore, while in the first to fifth examples one marker reagent part and plural reagent immobilization parts are adopted, the market reagent part is not necessarily provided in one position, and the device may be constituted by a combination of plural reagent immobilization parts and plural reagents. For example, when plural reagent immobilization parts are provided, a marker reagent part may be provided at the upper stream side of each reagent immobilization part. In this case, although the construction technique in manufacturing is complicated, an arbitrary number of marker reagent parts can be provided in arbitrary positions.

Further, the liquid samples to be measured include, for example, water, aqueous solution, bodily fluids such as urine, blood plasma, blood serum, and saliva, and solutions in which a solid, powder, or gas is dissolved, and the like, and applications of these samples include, for example, blood test, urine test, water examination, fecal examination, soil analysis, food analysis, and the like. Further, while the examples have been described with the C-reactive protein (CRP) as the target substance, the target substance may be antibody, immunoglobulin, hormone, protein and protein derivative such as enzyme and peptide, bacterium, virus, eumycetes, mycoplasma, parasite, infectious materials such as products or components of them, drugs such as curative drug and abused drug, tumor marker, and the like. To be specific, the target substance may be, for example, human chrionic gonadotropin (hCG), luteinizing hormone (LH), thyroid-stimulating hormone, follicular hormone, parathyroid hormone, adrenocorticotropic hormone, estradiol, prostate specific antigen, hepatitis B surface antigen, myoglobin, CRP, cardiac troponin, HbAlc, albumin, and the like. Furthermore, the above-mentioned examples can be executed for environmental analysis such as water examination and soil analysis, food analysis, and the like. Thereby, simple, speedy, highly sensitive and highly efficient measurement can be realized. Further, since anybody can perform measurement anytime and anywhere, it can be utilized as an analysis device for POCT.

APPLICABILITY IN INDUSTRY

An analysis device of the present invention can realize easy, speedy, highly sensitive, and highly efficient measurement when it analyzes and detects a target substance in a liquid sample on the basis of an arbitrary reaction such as an antigen-antibody reaction, and performs quantitation or semi-quantitation thereof. Further, since anybody can perform measurement anytime and anywhere, it is useful as an analysis device for POCT.

**Claims**

1. An analysis device for developing a liquid sample in a channel on an analysis element, and measuring a target substance in the liquid sample, comprising:

    a signal measurement unit for measuring a signal based on a reaction of the target substance in the liquid sample on the channel;
    a parameter collection unit for collecting a parameter which indicates a degree of influence on a measurement error of the target substance from the liquid sample developed on the channel;
    an algorithm holding unit for previously holding an algorithm comprising a relationship among the parameter, the signal, and a true value of the target substance; and
    an arithmetic processing unit for arithmetically processing an analysis value of the target substance from the signal on the basis of the parameter;
    wherein said arithmetic processing unit reads out the algorithm from the algorithm holding unit, and obtains, using the read algorithm, an analysis value of the target substance with the measurement error of the target substance being corrected on the basis of the parameter obtained in the parameter collection unit.

2. An analysis device as defined in Claim 1 wherein:

    said arithmetic processing unit includes a measurement value calculation unit for calculating a measurement value of the target substance in the liquid sample from the signal obtained in the signal measurement unit, and a measurement value correction unit for correcting the measurement value of the target substance so as to minimize the measurement error of the target substance, thereby obtaining an analysis value of the target substance; and
    said measurement value correction unit reads out an algorithm comprising a relationship between the parameter and the measurement error of the target substance from the algorithm holding unit, and corrects the measurement value of the target substance which is obtained in the measurement value calculation unit on the basis of the parameter obtained in the parameter collection unit by using the read algorithm, thereby obtaining an analysis value of the target substance.

3. An analysis device as defined in Claim 1 wherein said analysis element includes a sample application part for applying the liquid sample onto the channel, a marker reagent part which holds a marker reagent that reacts with the target substance so that the marker reagent can be eluted by development of the liquid sample, and a sample immobilization part which immobilizes and holds a reagent that comprehends the degree of the reaction between the target substance and the marker reagent.

4. An analysis device as defined in Claim 1 wherein said parameter is any of a developing speed, a developing time, and a developing distance which are obtained when the liquid sample is developed on the channel.

5. An analysis device as defined in Claim 1 wherein at least one of the signal measurement unit and the parameter collection unit uses electromagnetic radiation.

6. An analysis device as defined in Claim 1 wherein said analysis element is a dry type analysis element.

7. An analysis device as defined in Claim 1 wherein said signal measurement unit measures a signal based on a reaction that is derived from an antigen-antibody reaction.

8. An analysis device as defined in Claim 1 wherein said analysis element is an immunochromatography sensor.

9. An analysis device as defined in Claim 1 wherein said analysis element is a one-step immunochromatography sensor.

10. An analysis device as defined in Claim 1 wherein said channel comprises a monolayer or multilayer porous material.

11. An analysis device as defined in Claim 1 wherein:

    said algorithm holding unit holds a correction formula for correcting the measurement value of the target substance on the basis of the parameter; and
    said measurement value correction unit reads out the correction formula from the algorithm holding unit, and corrects the measurement value of the target substance using the read correction formula and the parameter, thereby obtaining an analysis value of the target substance.

**12.** An analysis device as defined in Claim 11 wherein:

said algorithm holding unit holds a plurality of said correction formulae;
said analysis device further includes an algorithm selection unit for selecting any of the plural correction formulae that are stored in the algorithm holding unit on the basis of the measurement value of the target substance; and said measurement value correction unit corrects the measurement value of the target substance using the correction formula selected by the algorithm selection unit and the parameter, thereby obtaining an analysis value of the target substance.

**13.** An analysis device as defined in Claim 1 wherein:

said algorithm holding unit holds a plurality of calibration curves for obtaining an analysis value of the target substance with the measurement error of the target substance being corrected, from the signal obtained in the signal measurement unit;
said analysis device further includes an algorithm selection unit for selecting any of the plural calibration curves that are stored in the algorithm holding unit on the basis of the parameter; and
said arithmetic processing unit obtains an analysis value of the target substance with the measurement error of the target substance being corrected, from the signal by using the calibration curve selected by the algorithm selection unit and the parameter.

**14.** An analysis device as defined in Claim 1 wherein said arithmetic processing unit obtains an analysis value of the target substance with the measurement error of the target substance due to an influence of the viscosity of the liquid sample, or the additive amount of the liquid sample, or the passage time after fabrication of the analysis element being corrected.

**15.** An analysis method for developing a liquid sample in a channel on an analysis element, and measuring a target substance in the liquid sample, comprising:

a parameter collection step of collecting a parameter which indicates a degree of influence on a measurement error of the target substance, from the liquid sample developed on the channel;
a signal measurement step of measuring a signal based on a reaction of the target substance in the liquid sample on the channel; and
an arithmetic processing step of reading out an algorithm from an algorithm holding unit which previously holds an algorithm comprising a relationship among the parameter, the signal, and a true value of the target substance, and obtaining, using the read algorithm, an analysis value of the target substance with the measurement error of the target substance being corrected on the basis of the parameter obtained in the parameter collection unit.

**16.** An analysis method as defined in Claim 15 wherein said arithmetic processing step includes a measurement value calculation step of calculating a measurement value of the target substance in the liquid sample from the signal obtained in the signal measurement step, and a measurement value correction step of correcting the measurement value of the target substance to obtain an analysis value of the target substance.

Fig.1

60 analysis device

parameter
collection
unit
30

arithmetic
processing unit
90

measurement
value
correction unit
50

measurement
value
calculation
unit
70

algorithm
holding
unit
40

signal
measurement
unit
20

analysis element
100

Fig.2

100

9

10

5

1

4  3  2

8

Fig.3

Fig.4

Fig.5

Fig.6(a)

Fig.6(b)

## Fig.7

liquid sample
developing direction

## Fig.8

detection of
analyte arrival

absorbance

time

Fig.9(a)

Fig.9(b)

Fig.9(c)

Fig.10

Fig.11

Fig.12(a)

Fig.12(b)

before correction

CRP concentration Log(mg/dL)

after correction

CRP concentration Log(mg/dL)

Fig.13(a)

Fig.13(b)

before correction

CRP concentration Log(mg/dL)

after correction

CRP concentration Log(mg/dL)

Fig.14

Fig.15

# Fig.16(a)

## without correction

degree of deviation (%)

CRP concentration (mg/dL)

# Fig.16(b)

## with correction

degree of deviation (%)

CRP concentration (mg/dL)

EP 1 909 103 A1

Fig.17

Fig.18

Fig.19

Fig.20

Fig.21

Fig.22(a)

analysis device 60

arithmetic processing unit 90

algorithm selection unit 80

algorithm holding unit 40

measurement value correction unit 50

liquid sample identification algorithm selection 130

liquid sample identification algorithms 110

parameter collection unit 30

signal measurement unit 20

measurement value calculation unit 70

measurement value correction algorithm selection 140

measurement value correction algorithms 120

analysis element 100

EP 1 909 103 A1

Fig.22(b)

analysis device 60

30 parameter collection unit

20 signal measurement unit

analysis element 100

arithmetic processing unit 90

algorithm selection unit 80

liquid sample identification algorithm selection 130

calibration curve selection 160

algorithm holding unit 40

liquid sample identification algorithms 110

calibration curves 150

Fig.22(c)

```
                              ( Apply liquid sample )
                                       │
                       ┌───────────────┴───────────────┐
                       ▼                               ▼
              ┌──────────────────┐           ┌──────────────────┐
              │ Measure signal from│           │ Collector parameter│
              │  analysis element  │           │ from analysis element│
              └──────────────────┘           └──────────────────┘
                       │                               │
                       │                               ▼
                       │                    ┌────────────────────┐       ┌────────────────────┐
                       │                    │ Select liquid sample│       │   Liquid sample    │
                       │                    │   identification    │◄──────│   identification   │
                       │                    │ algorithm by algorithm│       │ algorithms stored in│
                       │                    │   selection unit    │       │ algorithm holding unit│
                       │                    └────────────────────┘       └────────────────────┘
                       │                               │
                       │                               ▼
                       │                    ┌────────────────────┐
                       │                    │ Identify liquid sample│
                       │◄───────────────────│ (urine or blood plasma│
                       │                    │   or whole blood)   │
                       │                    └────────────────────┘
   ┌────────────────────┐        ▼
   │  Calibration curves │   ┌────────────────────┐
   │(for urine or blood plasma│ Select calibration curve│
   │ or whole blood)stored in│──►│ by algorithm selection unit│
   │  algorithm holding unit │   └────────────────────┘
   └────────────────────┘        │
                                 ▼
                        ┌────────────────────┐
                        │Calculate measurement value│
                        └────────────────────┘
                                 │
                                 ▼
              ┌────────────────────────────────┐       ┌────────────────────┐
              │ Select measurement value correction│       │Measurement value correction│
              │  algorithm by algorithm selectioin unit│◄──────│  algorithms stored in │
              └────────────────────────────────┘       │ algorithm holding unit│
                                 │                      └────────────────────┘
                                 ▼
              ┌────────────────────────────────┐
              │ Substitute parameter and measurement value into│
              │ selected measurement value correction algorithm│
              └────────────────────────────────┘
                                 │
                                 ▼
                        ( Analysis value )
```

EP 1 909 103 A1

## Fig.23

## Fig.24

Fig.25

before correction

after correction

## INTERNATIONAL SEARCH REPORT

International application No.

PCT/JP2006/314001

A.  CLASSIFICATION OF SUBJECT MATTER
*G01N33/52*(2006.01)i, *G01N21/78*(2006.01)i, *G01N33/543*(2006.01)i

According to International Patent Classification (IPC) or to both national classification and IPC

B.  FIELDS SEARCHED

Minimum documentation searched (classification system followed by classification symbols)
G01N1/00-37/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y/A | WO 1999/060391 A1 (Arkray, Inc.), 25 November, 1999 (25.11.99), Full text & US 6576117 B1 & EP 1081490 A1 | 1-3,5-16/4 |
| Y/A | JP 8-075748 A (Mochida Pharmaceutical Co., Ltd.), 22 March, 1996 (22.03.96), Claims & US 5723345 A & EP 690306 A1 & CA 2152756 A | 1-3,5-16/4 |

☒  Further documents are listed in the continuation of Box C.  ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 August, 2006 (28.08.06) | 12 September, 2006 (12.09.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/314001

C (Continuation).    DOCUMENTS CONSIDERED TO BE RELEVANT

| Category* | Citation of document, with indication, where appropriate, of the relevant passages | Relevant to claim No. |
|---|---|---|
| Y/A | WO 2002/025253 A1  (Matsushita Electric Industrial Co., Ltd.),<br>28 March, 2002 (28.03.02),<br>Claims<br>& US 2003/054567 A1    & EP 1249696 A1<br>& KR 2002070447 A | 1-3,5-16/4 |
| Y | WO 2003/056312 A1  (Arkray, Inc.),<br>10 July, 2003 (10.07.03),<br>Claims<br>& US 2005/107956 A1    & EP 1460414 A1 | 13 |
| Y | JP 2001-091512 A  (Matsushita Electric Industrial Co., Ltd.),<br>06 April, 2001 (06.04.01),<br>Par. Nos. [0031], [0032]<br>(Family: none) | 13 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3586743 B
- JP 2000262298 A
- JP 2001091512 A
- JP HEI875748 B